# FASCICULE DE BREVET EUROPEEN

(11) **EP 2 939 029 B1**
(45) Date de publication et mention de la délivrance du brevet: **10.10.2018**
(21) Numéro de dépôt: 13821895.3
(22) Date de dépôt: 27.12.2013
(51) Int. Cl.: G01N 33/82, C07D 475/04

(54) **DÉRIVÉS DE FOLATE, PARTICULIÈREMENT UTILES DANS LE CADRE DU DOSAGE DE FOLATE(S)**
FOLATDERIVATE, INSBESONDERE IM KONTEXT DES FOLATTESTS
FOLATE DERIVATIVES, USEFUL IN PARTICULAR IN THE CONTEXT OF THE FOLATE ASSAY

(30) Priorité: 27.12.2012 FR 1262898
(43) Date de publication de la demande: 04.11.2015
(73) Titulaire: bioMérieux, 69280 Marcy-l'Étoile (FR)
(72) Inventeur: GUO, Yuping, 69170 Tarare (FR); CHEUCLE, Sylvie, 69890 La Tour de Salvagny (FR)
(74) Mandataire: Murgitroyd & Company
(86) Numéro de dépôt international: PCT/FR2013/053271
(87) Numéro de publication internationale: WO 2014/102511

(56) Documents cités:
- EP-A2- 1 273 917
- WO-A1-02/085908
- FR-A1- 2 455 602
- William B Wright ET AL: "Analogs of Pteroylglutamic Acid. IV. Replacement of Glutamic Acid by Other Amino Acids", J. Am. Chem. Soc., 1 septembre 1949 (1949-09-01), XP055084199, Extrait de l'Internet: URL:http://pubs.acs.org/doi/pdf/10.1021/ja 01177a019 [extrait le 2013-10-16]
- WEI HUANG ET AL: "Homogeneous Bioluminescence Competitive Binding Assay for Folate Based on a Coupled Glucose-6-phosphate Dehydrogenase-Bacterial Luciferase Enzyme System", ANALYTICAL CHEMISTRY, vol. 68, no. 9, 1 janvier 1996 (1996-01-01), pages 1646-1650, XP055109379, ISSN: 0003-2700, DOI: 10.1021/ac950757m
- MULLER C ET AL: "Organometallic <99m>Tc-technetium(I)- and Re-rhenium(I)-folate derivatives for potential use in nuclear medicine", JOURNAL OF ORGANOMETALLIC CHEMISTRY, ELSEVIER-SEQUOIA S.A. LAUSANNE, CH, vol. 689, no. 25, 6 décembre 2004 (2004-12-06), pages 4712-4721, XP004656577, ISSN: 0022-328X, DOI: 10.1016/J.JORGANCHEM.2004.08.045
- "Abbott Axsym System", , 1 juillet 2010 (2010-07-01), pages 1-10, XP055084042, Extrait de l'Internet: URL:http://www.ilexmedical.com/files/PDF/F olate_AXS.pdf [extrait le 2013-10-15]

## Description

### Domaine technique

La présente invention concerne des dérivés de folate, notamment utiles pour doser un/des folate(s) dans un échantillon biologique, *in vitro,* de préférence en mettant en oeuvre des techniques d'immunodosage par compétition non radio-isotopiques.

### Etat de la technique

La vitamine B9 est la dénomination générique donnée à une famille de composés chimiquement et biologiquement très voisins, dérivant tous de l'acide folique. Une des caractéristiques principales de ces composés est que leur présence en quantité insuffisante, voire leur absence, provoque une anémie chez l'homme ou l'animal. La famille des folates comprend, entre autres, la vitamine M, la vitamine Bc, la folacine et l'acide folique. Au sens de la présente demande, chacun des composés appartenant à cette famille est dénommé « folate », plusieurs membres de cette famille sont désignés en tant que « folates », alors que le mélange des constituants de la famille de la vitamine B9 est dénommé « folate total ».

Comme cela est bien connu de l'homme du métier, l'acide folique, appelé aussi acide ptéroyl-monoglutamique, est formé d'un groupement ptérine, d'un groupement p-aminobenzoïque et d'un groupement glutamate comme représenté ci-après, sur la formule générale (G) : 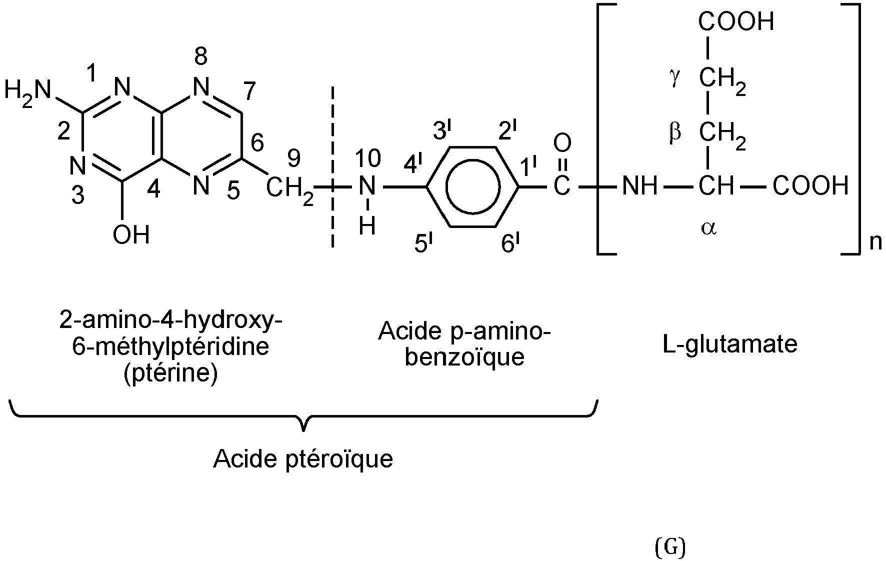 dans laquelle n est le nombre entier 1.

Comme représenté sur cette formule générale, l'acide folique possède deux fonctions acide carboxylique sur sa partie glutamate, l'une en position a, l'autre en position γ.

Dans l'alimentation, les folates se trouvent majoritairement sous forme méthyl- ou formyl-polyglutamates réduits. Lors de la digestion, ces polyglutamates sont transformés en monoglutamates, activement absorbés par les entérocytes. Ensuite, les monoglutamates sont transformés en 5-méthyltétrahydrofolate (5-MTHF), forme sous laquelle les folates traversent la barrière intestinale et atteignent la circulation systémique.

Dans le sang, la majeure partie des folates circulants est liée avec une faible affinité à diverses protéines : l'α2-macroglobuline (40%), l'albumine (33%) et la transferrine (23%). Les concentrations plasmatiques en vitamine B9 varient de 5 à 15 µg/L et sont fortement impactées par les apports alimentaires. La concentration en vitamine B9 est environ 20 fois plus élevée dans les hématies qui peuvent contenir jusqu'à 95% des folates circulants. De multiples formes de folates sont présentes dans le sérum humain, mais la forme circulante et intracellulaire prépondérante est le 5-méthyltétrahydrofolate (5-MTHF), qui est aussi la forme de stockage hépatique. De façon générale, les composés biologiquement actifs sont uniquement les formes réduites : le dihydrofolate (DHF), et surtout le tétrahydrofolate (THF) ainsi que ses dérivés méthylés ou formylés. Tel qu'indiqué précédemment, au sens de la présente demande, la dénomination « folates » recouvre notamment ces formes réduites ; chacune desdites formes, prise séparément, étant dénommée « folate ».

Les cellules eucaryotes, ainsi que certaines cellules procaryotes sont incapables de synthétiser l'acide folique. Elles utilisent donc des systèmes de transport transmembranaire qui permettent d'internaliser la molécule exogène. A l'heure actuelle, deux principaux systèmes de transport ont été décrits. Toutefois, il existe probablement également des voies secondaires comme la diffusion passive. Les folates oxydés comme l'acide folique sont transportés à l'intérieur des cellules par les récepteurs des folates (FR), « folate receptors » en langue anglaise (Antony, 1992 [1]). Ces protéines étaient anciennement dénommées « folate binding proteins » (FBPs). Trois isoformes ont été identifiées chez l'homme, dénommées respectivement FRa (P15328), PRβ (P14207) et FRγ (P41439), le code indiqué entre parenthèses correspondant à l'identifiant de la protéine dans la base de données UniProt (http://www.uniprot.org). Les FRa et β sont ancrés dans la membrane plasmatique par une partie lipidique, le glycosylphosphatidylinositol (GPI). L'isoforme γ est sécrété. Les folates réduits sont, quant à eux, transportés par une protéine dénommée transporteur des folates réduits (P41440) ou « reduced folate carrier » en langue anglaise (RFC). Il s'agit d'une protéine membranaire intégrale fortement glycosylée qui possède plusieurs domaines transmembranaires.

De par leur structure chimique, les folates jouent un rôle essentiel dans la synthèse et le métabolisme des constituants de base de notre organisme, à savoir les acides aminés et les bases (purines et pyrimidines).

Le THF, coenzyme essentielle, est capable de fixer et de céder des radicaux à un atome de carbone. Il est impliqué dans la synthèse de la glycine et le catabolisme de l'histidine.

Le 5-MTHF permet la reméthylation de l'homocystéine en méthionine via la méthylcobalamine et la méthionine synthéase. Les folates interviennent aussi dans plusieurs étapes clef des biosynthèses des purines et des pyrimidines, conditionnant ainsi la synthèse des acides nucléiques ADN et ARN. A cause de ce rôle central, une carence en folate(s) a de lourdes conséquences et de nombreuses manifestations physiopathologiques.

La carence profonde en folates donne lieu à des signes généraux, hématologiques et neuropsychiatriques. Lentement, une asthénie et une anorexie apparaissent. L'anémie peut être précédée par une macrocytose isolée. Cette anémie, souvent de type mégaloblastique, est une des manifestations les plus fréquentes de la carence en folates. En outre il existe souvent une carence combinée en folates et en fer qui se traduit, au lieu de la classique anémie macrocytaire, par une anémie normocytaire avec une présence de corps de Jolly sur le frottis. Cette anémie est due au fait que les purines et pyrimidines ne sont pas disponibles en quantité suffisante, résultant ainsi en l'impossibilité pour les cellules souches sanguines de synthétiser du matériel génétique et donc de se diviser. En revanche, les cellules existantes continuent à grandir, ce qui explique en partie le type généralement mégaloblastique de l'anémie liée à une carence en folates.

Les folates sont également nécessaires pour le bon fonctionnement du cerveau et contribuent à la santé mentale et l'équilibre émotionnel. Ainsi, une carence en vitamine B9 induit des troubles neuropsychiatriques. Ces troubles pourraient en partie être liés à des anomalies de synthèse de certaines amines et de la glycine. Cette dernière est aussi un neuro-transmetteur.

Par leur contribution à la synthèse du matériel génétique, un apport satisfaisant en folates est particulièrement nécessaire lors de l'enfance, l'adolescence et la grossesse. En effet, du retard dans le développement psychomoteur et une hypotrophie staturo-pondérale sont souvent retrouvés chez les enfants ayant des carences en folates. Pendant la grossesse, une carence en folate(s) peut induire un retard ou des anomalies dans le développement du foetus, voire des malformations congénitales comme la spina bifida qui est une fermeture incomplète du tube neural ou encore la trisomie.

La carence en folates est également associée à un risque accru de maladies cardiovasculaires, plus précisément de thromboses artérielles et/ou veineuses et d'arthérosclérose. Le risque est lié à l'augmentation du taux plasmatique d'homocystéine, résultant du défaut de méthylation de ce composé en méthionine.

Cette liste n'est pas limitative et la carence en folates est susceptible d'induire d'autres affections/pathologies. Il est donc primordial de pouvoir doser tout ou partie des folates chez un individu humain ou animal, de préférence le folate « total », à savoir constitué par le mélange des différentes formes folates.

En outre, il est également important de pouvoir quantifier tout ou partie des folates présents au sein d'échantillons d'origine alimentaire (destinés à l'alimentation humaine ou animale) afin de vérifier l'apport en vitamine B9 des aliments d'intérêt. Il peut également s'avérer intéressant de doser les folates dans les produits de type compléments alimentaires, afin de s'assurer que ces derniers contiennent une quantité/concentration suffisante en folate(s). De tels compléments alimentaires peuvent notamment servir à prévenir d'éventuelles carences en vitamine B9 .

D'un point de vue clinique, le dosage des folates dans le sang total, le sérum, le plasma, ou dans les érythrocytes présente un intérêt certain. La diminution de la concentration sanguine en folates traduit potentiellement une carence qu'il convient d'explorer sur le plan clinique, éventuellement en y associant d'autres dosages vitaminiques ou de métabolites. Le taux sanguin des folates est soumis à des variations en fonction du régime alimentaire ou des prises de médicaments. C'est le taux de folates érythrocytaires qui représente la meilleure estimation des réserves en folates de l'organisme.

Il existe plusieurs méthodes permettant la quantification plasmatique, sérique et/ou érythrocytaire des folates dans des échantillons biologiques d'origine clinique, à savoir provenant de patients. Ces méthodes peuvent être classées en trois groupements principaux, à savoir : (1) les techniques microbiologiques, (2) les techniques chromatographiques et (3) les immunodosages par compétition.

Les techniques microbiologiques (1) utilisent généralement un germe « folates-dépendant », dont la croissance est proportionnelle au taux de vitamine présent dans l'échantillon à doser. En général, les échantillons sont déprotéinisés à 100°C en présence de vitamine C, qui joue le rôle d'anti-oxydant. La mise en contact avec la souche bactérienne est réalisée pendant 20 heures à 37°C. Le germe le plus fréquemment utilisé, *Lactobacillus casei* est sensible à toutes les formes oxydées et réduites de folates ; d'autres germes sont sensibles à des formes plus spécifiques. Par exemple, *Streptococcus faecalis* permet de doser toutes les formes de folates à l'exception du 5-MTHF. La concentration en 5-MTHF, forme prépondérante dans le sérum et les érythrocytes, est obtenue par différence entre les valeurs de *L. casei* et de *S. faecalis.* Un troisième germe, *Pediococcus cerevisiae* est sensible exclusivement au N5- formyl- THF (acide folinique).

Ces techniques microbiologiques (1), bien que généralement sensibles et reproductibles, sont fastidieuses et chronophages. En outre, elles présentent des risques d'interférence avec les antibiotiques et les antimitotiques, tels que le méthotrexate, la triméthoprime et la pyriméthamine.

Les dosages de type chromatographique (2) permettent la séparation des différents composés appartenant à la famille des folates. A titre d'exemples de dosage par chromatographie, l'on peut notamment citer :
- le dosage par chromatographie sur couche mince couplée à l'HPLC (Reif, V. D. et al., 1977 [2]),
- le dosage par chromatographie (généralement en phase gazeuse ou liquide) couplée à de la spectrométrie de masse (MS), par exemple par :
   a) chromatographie en phase gazeuse/spectrométrie de masse avec dilution isotopique (ID-GCMS) (Dueker, S. R. et al., 2000 [3]), ou
   b) chromatographie en phase liquide - spectrométrie de masse en tandem avec dilution isotopique (ID-LC-MS/MS) (Pfeiffer, C. M. et al., 2004 [4]).

Ces dosages de type chromatographique (2) ont notamment pour inconvénient de nécessiter la mise au point de tests très techniques, requérant un personnel qualifié. L'instrument s'avère en outre couteux.

Eu égard aux problématiques rencontrées lors de la mise en oeuvre des techniques microbiologiques (1) et des techniques chromatographiques (2) (cf. supra), les immunodosages par compétition (3) ont été développés. Tout en réduisant le temps d'analyse, ces derniers (3) permettent de doser le folate « total » et donc de poser un diagnostic clinique fiable par rapport à une éventuelle carence en folates.

Ces procédés d'immunodosage (3), également appelés dosages immunologiques ou tests immuno-chimiques, impliquent la liaison de l'analyte à détecter - en l'espèce le/les folate(s) - avec au moins un premier composé qui est un partenaire de liaison à cet analyte. Comme le dosage du ou des folate(s) se fait par compétition, le procédé implique également au moins un deuxième composé qui entre en compétition avec le folate à doser vis-à-vis de la fixation sur le partenaire de liaison, ce deuxième composé étant un dérivé de folate. Le suivi de cette réaction implique le marquage d'un des deux composés. On appelle ce composé marqué, conjugué marqué ou traceur.

Bien entendu, le préfixe « immuno », par exemple dans « immunodosage », n'est pas à considérer dans la présente demande comme indiquant strictement que le partenaire de liaison est un partenaire immunologique, tel qu'un anticorps ou un fragment d'anticorps. En effet, comme cela est bien connu de l'homme du métier, ce terme est plus largement utilisé pour désigner des tests et des procédés dans lesquels le partenaire de liaison, également dénommé ligand, n'est pas un partenaire immunologique mais consiste, par exemple, en un récepteur de l'analyte que l'on souhaite doser. La condition étant que le partenaire de liaison soit capable de se lier à l'analyte, de préférence de manière spécifique. Ainsi, il est connu de parler du dosage ELISA (Enzyme-Linked Immunosorbent Assay) pour des dosages qui utilisent des partenaires de liaison non immunologiques *stricto sensu,* appelés plus largement en anglais « Ligand Binding Assay », que l'on pourrait traduire en français par « Dosage utilisant la liaison à un ligand », alors que le terme « immuno » est inclus dans l'acronyme ELISA. Dans un souci de clarté et d'uniformité, le terme « immuno » est employé dans la présente demande pour désigner tout dosage utilisant un partenaire de liaison adapté pour se lier à l'analyte à quantifier, de préférence de manière spécifique, même quand ce partenaire de liaison n'est pas de nature ou d'origine immunologique au sens strict.

Dans le cadre des immunodosages par compétition (3), et lorsque l'on utilise un conjugué marqué, l'on distingue trois types d'immunodosage par compétition en fonction de la nature du conjugué marqué et du type de signal émis par ledit conjugué, à savoir:
- les immunodosages radio-isotopiques (Waxman S. et Schreiber C., 1980 [5]),
- les dosages immuno-enzymatiques ou EIA « enzyme linked immunoassay - assay » ; en fonction du substrat enzymatique choisi, le signal peut être de type colorimétrique (Hansen, S. I. et Holm, J., 1988 [6]), de type fluorescence ou chemiluminescent,
- les immunodosages électrochemiluminescentes (Owen, W.E. et Roberts, W. L.2003 [7]).

Les deux derniers types d'immunodosage par compétition sont dénommés « immunodosages par compétition non radio-isotopiques ».

Le développement des méthodes radio-isotopiques (RIA), à partir des années 1960, a révolutionné la quantification des vitamines et notamment de la vitamine B9.

La demande de brevet WO 80/00562 illustre ceci en divulguant des dérivés radioactifs du folate, substitués au niveau de la fonction acide carboxylique portée par le carbone α et/ou par le carbone γ du dérivé glutamate. Le marquage radioactif provient de l'insertion d'iode-125 ou 130 au sein du noyau phénol d'un motif tyrosine.

La demande de brevet français FR-A-2455602 concerne également l'obtention de composés radio-iodés et mentionne des dérivés d'acide ptéroïque de formule générale : 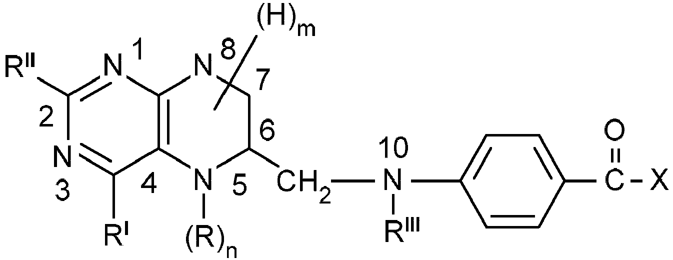 dans laquelle le groupement glutamate est remplacé par le radical X, ce radical X représentant, au choix, le reste d'un amino-acide ou d'un « des-carboxyamino-acide » renfermant obligatoirement un noyau aromatique ou hétérocyclique, indispensable au marquage radio-isotopique (à l'iode 125, 131 ou 123). Ce noyau aromatique ou hétérocyclique iodé est séparé du groupement acide p-aminobenzoïque par une chaîne ne comportant pas plus de 5 atomes de carbone, reliée au groupement acide p-aminobenzoïque par une amine secondaire. A titre de « reste de des-carboxyamino-acides » renfermant un noyau aromatique ou hétérocyclique, les structures suivantes sont divulguées :

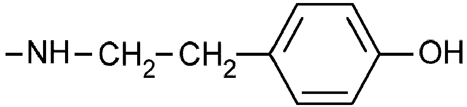

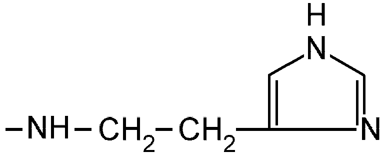

Toutefois, ces dosages radio-isotopiques (RIA) avaient notamment pour inconvénient le traitement des déchets radioactifs et la durée de demi-vie relativement courte des réactifs marqués relativement courte.

C'est pourquoi les immunodosages par compétition non radio-isotopiques se sont développés au détriment du RIA que l'on n'utilise que rarement aujourd'hui.

A titre illustratif, la publication Arcot J. et al, 2005 [8] décrit un procédé de dosage de l'acide folique par liaison à une protéine marquée par une enzyme (« enzyme-labelled protein binding assay » en langue anglaise), ladite protéine marquée étant la FBP, et le procédé étant basé sur la compétition entre les molécules d'acide folique libres dans l'échantillon biologique et celles préalablement immobilisées sur une plaque de microtitration pour se fixer sur les FBPs marquées. Après rinçage, l'étape de révélation s'effectue en introduisant un substrat enzymatique incolore, induisant une coloration bleue après clivage par l'enzyme fixée à la FBP. Comme cela est généralement le cas dans les dosages par compétition, la quantité d'acide folique libre présent dans l'échantillon est déterminée en référence à une courbe de calibration, à partir de laquelle la quantité d'acide folique libre présente dans l'échantillon est déduite en fonction de l'intensité lumineuse mesurée.

Le kit Abbott Axsym Folate (Cat. No. B7K460, Abbott Laboratories) permet également la mise en oeuvre d'un procédé de dosage de folates par compétition. Ce kit utilise, comme partenaire de liaison, la protéine FBP et, comme conjugué marqué, l'acide ptéroïque (un analogue de folate) couplé à la phosphatase alcaline (PAL). Le principe du test est basé sur la compétition entre l'acide folique à doser et le susdit conjugué dans le cadre de la fixation à la FBP soluble. A la suite de la réaction de compétition, la FBP est mise en présence d'anticorps monoclonaux anti-FBP et la réaction antigène-anticorps a lieu. Lesdits anticorps sont couplés de manière covalente à la carboxyméthylamylose, un polyanion. Ainsi, les complexes sont capturés par interactions électrostatiques polyanion-polycation, sur une matrice chargée positivement. Toutefois, la Demanderesse a découvert que la sensibilité de ce dosage n'était pas pleinement satisfaisante, notamment en ce qui concerne la quantification de faibles concentrations en folates.

EP1273917 décrit des dérivés de folate et leur utilisation dans le dosage de folate dans des échantillons biologiques. Certains dérivés portent un groupement carboxylique en position alpha., d'autres dérivés n'ont pas de groupement méthylène entre le groupement "ptérine" et le groupement acide p-amino-benzoïque.

Il existe donc un besoin urgent d'améliorer la sensibilité analytique des immunodosages non radio-isotopiques du folate afin d'obtenir un procédé utilisable dans les échantillons d'origine clinique (échantillons biologiques) et/ou dans les échantillons d'origine agroalimentaire contenant une faible concentration en folate.

### Exposé de l'invention

La Demanderesse a découvert, contre toute attente, de nouveaux dérivés du folate qui permettent de pallier tout ou partie des inconvénients mentionnés supra, en ce sens que leur utilisation dans un dosage par compétition non radio-isotopique (tel qu'un immunodosage), notamment en tant que traceur, permet d'obtenir une sensibilité analytique accrue, en particulier dans les gammes de concentration en folate les plus faibles.

Ainsi, est décrite l'utilisation d'un dérivé de folate pour doser le/les folate(s) *in vitro* dans un échantillon, tel qu'un échantillon biologique, ledit dosage étant un immunodosage par compétition non radio-isotopique et ledit dérivé de folate étant décarboxylé en position α. Ladite position α est telle que représentée sur la formule générale (G) susvisée.
En effet, la Demanderesse a notamment découvert, de manière surprenante, que les dérivés du folate décarboxylés en position α permettaient d'améliorer de manière significative la sensibilité analytique des immunodosages par compétition non radio-isotopiques permettant le dosage *in vitro* du/des folate(s).

De préférence, le dérivé de folate selon l'invention est différent des composés NSP-DMAE-HD-ptéroate et NSP-DMAE-HEG-ptéroate représentés respectivement par les formules (A) et (B) infra :

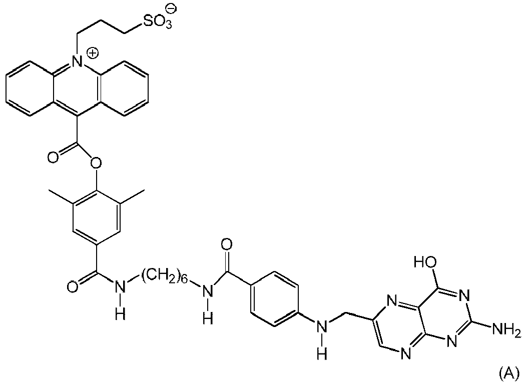

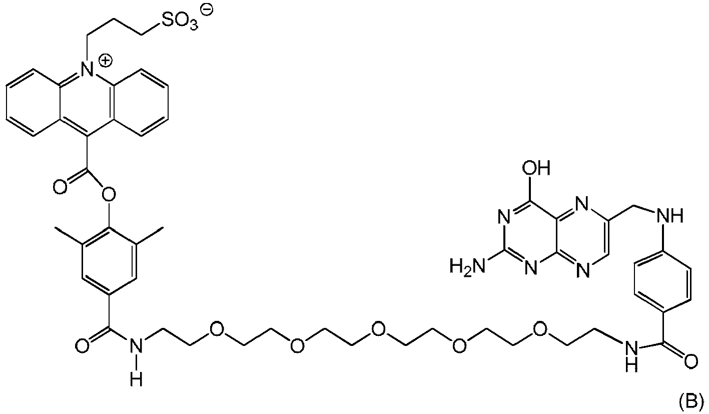

De manière avantageuse, le dérivé de folate selon l'invention ne comprend pas de motif -(O-CH₂-CH₂)- et est différent du composé NSP-DMAE-HD-ptéroate représenté par la formule (A) supra.

Tel qu'indiqué précédemment, le préfixe « immuno », par exemple au sein du terme « immunodosage », ne doit pas être interprété étroitement comme désignant un partenaire de liaison de nature et/ou d'origine immunologique, tel qu'un anticorps. En effet, le partenaire de liaison utilisé dans le test immunologique par compétition peut tout à fait être, par exemple, un récepteur de l'analyte que l'on souhaite doser, en l'espèce le récepteur des folates. Les techniques d'immunodosage non radio-isotopiques applicables selon la présente invention peuvent être toutes techniques connues de l'homme du métier employant un partenaire de liaison se liant avec une affinité suffisante au(x) folate(s) pour que le dosage par compétition soit correctement effectué.

Selon un mode de réalisation préféré, le dérivé de folate selon l'invention est utilisé pour doser une pluralité de folates, voire, de préférence, le folate total (tel que défini supra).

Selon un mode de réalisation particulier, le dérivé de folate selon l'invention est utilisé pour doser l'acide folique (acide ptéroyl-monoglutamique).

Les termes « doser » et « dosage » se rapportent, dans la présente demande, à la détermination d'une quantité/concentration du ou des analyte(s) d'intérêt, à savoir du/des folate(s).

Est également décrite l'utilisation d'un dérivé de folate pour doser le folate *in vitro* dans un échantillon tel qu'un échantillon biologique, ledit dosage étant un immunodosage par compétition, de préférence non radio-isotopique, ledit dérivé de folate répondant à la formule générale (I) : 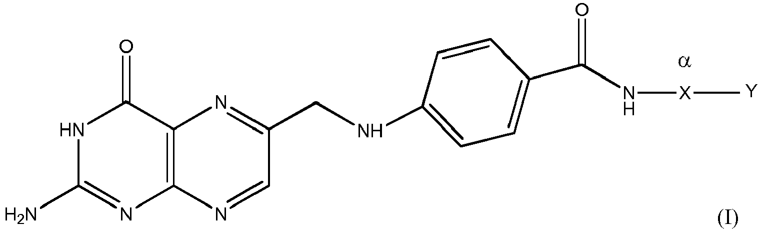 dans laquelle :
- X est une chaîne hydrocarbonée aliphatique comprenant de 1 à 10 atomes de carbone dans laquelle le carbone placé en position α ne porte de fonction acyle telle qu'une fonction acide carboxylique ;
- Y représente un groupement fonctionnel adapté pour permettre le couplage avec une molécule distincte M, telle qu'une protéine, ledit couplage comprenant la formation d'au moins une liaison covalente entre Y et un groupement fonctionnel porté par ladite molécule distincte M.

L'invention a pour objet l'utilisation d'un dérivé de folate pour doser le/les folate(s) *in vitro* dans un échantillon, tel qu'un échantillon biologique, ledit dosage étant un immunodosage par compétition non radio-isotopique, et ledit dérivé de folate répondant à la formule générale (I) : 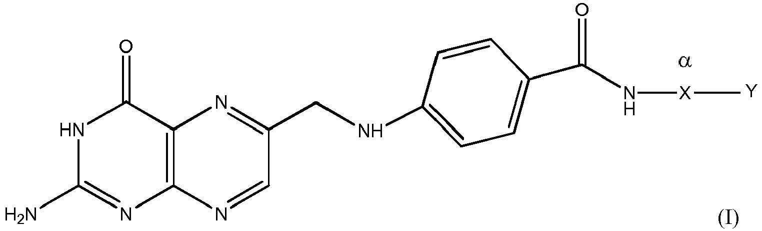 dans laquelle :
- X est une chaîne hydrocarbonée aliphatique contenant de 1 à 10 atomes de carbone, ladite chaîne hydrocarbonée aliphatique ne contenant que de l'hydrogène et du carbone ; et
- Y représente un groupement fonctionnel adapté pour permettre le couplage avec une molécule distincte M, telle qu'une protéine, ledit couplage comprenant la formation d'au moins une liaison covalente entre Y et un groupement fonctionnel porté par ladite molécule distincte M, Y étant un groupement de type centre électrophile ou un groupement de type centre nucléophile, de préférence un groupement de type centre électrophile, adapté pour permettre la formation d'une liaison amide, ester, ou thioester, de préférence amide ou ester, avantageusement amide, entre Y et le groupement fonctionnel porté par ladite molécule distincte M.

Par « chaîne hydrocarbonée aliphatique », l'on entend, au sens de la présente invention, une chaîne hydrocarbonée linéaire ou ramifiée ouverte (acyclique). Conformément à la définition communément admise et présentée dans les ouvrages de référence, une chaîne hydrocarbonée doit bien évidemment être comprise, au sens de la présente invention, comme ne contenant que de l'hydrogène (H) et du carbone (C). En d'autres termes, la chaîne hydrocarbonée aliphatique X est uniquement fonctionnalisée par le groupement fonctionnel Y susvisé et ne contient, par définition, pas d'hétéroatome (tel que l'oxygène, l'azote, le souffre, le phosphore, les halogènes etc.).

La combinaison de la déacylation (dont la décarboxylation est un exemple) en position α du dérivé de folate selon l'invention et d'une chaîne hydrocarbonée aliphatique comprenant de 1 à 10 atomes de carbone résulte en des dérivés de folate permettant l'obtention d'une excellente sensibilité analytique lors de leur mise en oeuvre dans des immunodosages par compétition (de préférence non radio-isotopiques).

Ladite molécule M est, par exemple une molécule marqueur Mm. Cette molécule M peut également consister en un bras chimique ou « linker ».

De préférence, X est une chaîne hydrocarbonée comprenant de 2 à 7 atomes de carbone, de préférence de 3 à 5 atomes de carbone, avantageusement X est une chaîne hydrocarbonée de 3 atomes de carbone ou de 5 atomes de carbone.

Avantageusement, X est une chaîne hydrocarbonée saturée.

De préférence, X est une chaîne hydrocarbonée linéaire.

Selon un mode de réalisation particulièrement préféré, X est une chaîne hydrocarbonée aliphatique, linéaire et saturée, comprenant un nombre d'atomes de carbone tel que défini précédemment. En d'autres termes, et selon ce mode de réalisation particulièrement préféré, le dérivé de folate selon l'invention peut être représenté par la formule générale (I') suivante : 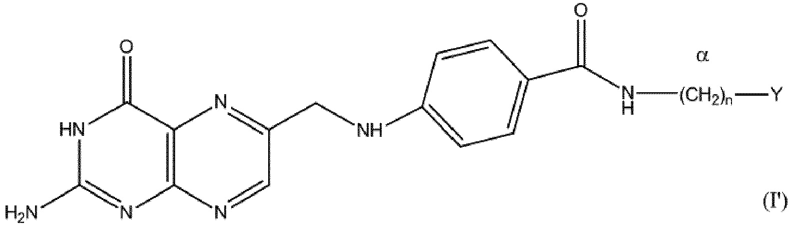 dans laquelle :
- n est un nombre entier compris entre 1 et 10. Avantageusement, n est un nombre entier compris entre 2 et 7, de préférence entre 3 et 5, avantageusement n représente le nombre entier 3 ou 5.
- Y est tel que défini supra.

Tel qu'indiqué précédemment, Y est un groupement de type centre électrophile ou un groupement de type centre nucléophile, de préférence un groupement de type centre électrophile, adapté pour permettre la formation d'une liaison amide, ester, ou thioester, de préférence amide ou ester, avantageusement amide, entre Y et le groupement fonctionnel porté par ladite molécule distincte.

Selon un mode de réalisation préféré, Y est un groupement de type centre électrophile, répondant à la formule générale (II) suivante : 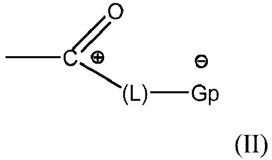 dans laquelle Gp est un groupement partant, éventuellement relié à la fonction carbonyle par un bras L, Gp étant adapté pour être dissocié du groupement de type centre électrophile lors d'une réaction avec un groupement nucléophile porté par ladite molécule distincte M, tel qu'une amine primaire.

La présence du bras L est donc facultative dans le composé de formule générale (II), raison pour laquelle ce bras L est représenté entre parenthèses dans ladite formule. Ainsi, aux fins de la présente demande, le groupement de formule (L)-Gp peut donc désigner un groupement de formule L-Gp (présence du bras L) ou un groupement partant Gp (absence dudit bras L).

De préférence, ladite réaction du groupement de type centre électrophile de formule générale (II) avec ledit groupement nucléophile porté par la dite molécule M - tel qu'une amine primaire - est une réaction de substitution nucléophile.

Toujours dans le mode de réalisation préféré selon lequel Y est un groupement de type centre électrophile, le groupement (L)-Gp est choisi parmi les groupements adaptés pour permettre la formation d'une liaison amide, ester, ou thioester, de préférence amide ou ester, avantageusement amide, entre ledit groupement de type centre électrophile et un groupement fonctionnel porté par ladite molécule distincte M. De préférence, ce dernier est un groupement nucléophile tel qu'une amine primaire.

Avantageusement, le groupement (L)-Gp est choisi parmi : -OH, -NH-(CH₂)ₘ-COOH,-N₃, 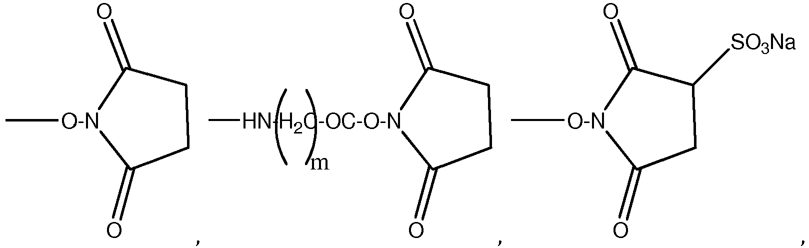 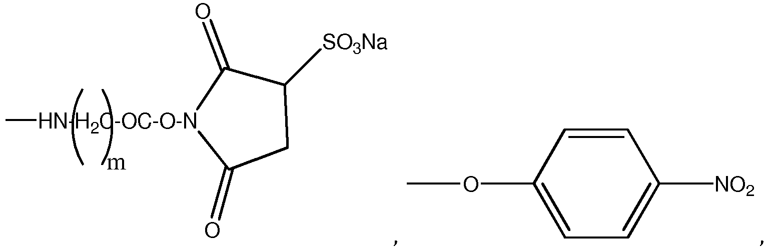 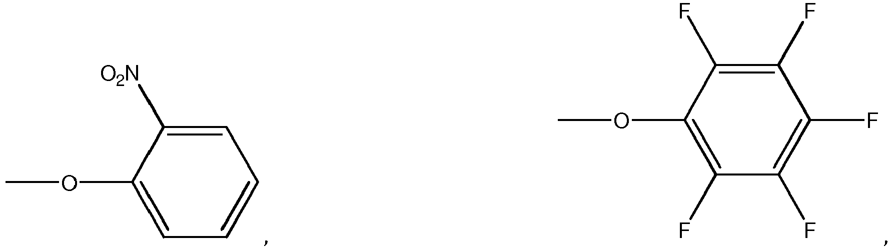 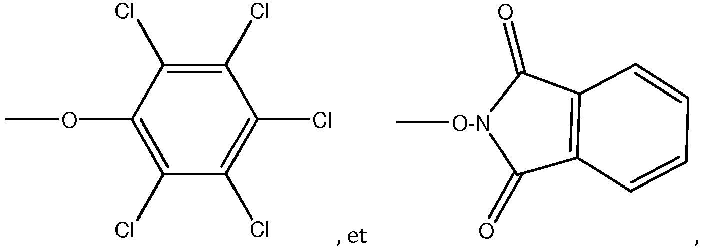 m étant un nombre entier compris entre 1 et 10.

Parmi les groupements (L)-Gp susvisés, l'homme du métier parviendra à distinguer, sans difficultés excessives, les groupements partants Gp (tels que les groupements-OH, -N₃, et -N-oxy-succinimide) des groupements L-Gp, à savoir notamment :

-NH-(CH₂)ₘ-COOH,

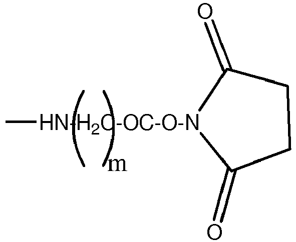

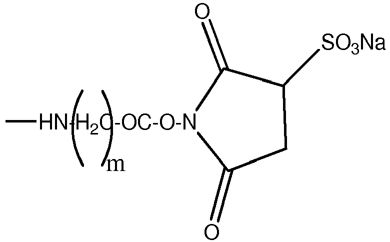

au sein desquels le bras L est constitué par la partie -NH-(CH₂)ₘ-CO (ou -NH-(CH₂)ₘ-OC).

A des fins de clarté, l'on notera que le groupement dénommé ci-dessus « -N-oxy-succinimide » est le groupement de formule suivante :

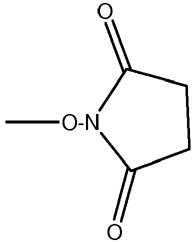

L'utilisation de tels groupements (L)-Gp permet de favoriser la formation d'une liaison amide, ester, ou thioester, de préférence amide ou ester, avantageusement amide, entre le groupement de type centre électrophile Y et un groupement fonctionnel porté par la molécule distincte M. Ces groupements (L)-Gp sont particulièrement adaptés pour permettre la formation d'une liaison amide entre le susdit groupement de type centre électrophile et une fonction amine - telle qu'une amine primaire - portée par la molécule M.

De préférence, m est compris entre 1 et 5, avantageusement entre 1 et 3.

Avantageusement, Y est un groupement de type centre électrophile de formule générale (II) et le groupement (L)-Gp est choisi parmi :
-OH et -NH-(CH₂)ₘ-COOH et 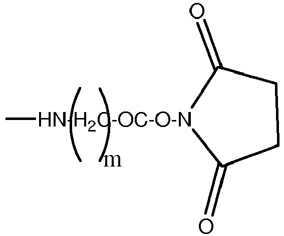
, m étant compris entre 1 et 10 et de préférence m étant égal à 1.

Selon un mode de réalisation particulièrement préféré, Y est un groupement de type centre électrophile de formule générale (II) et (L)-Gp est le groupement L-Gp de formule suivante :

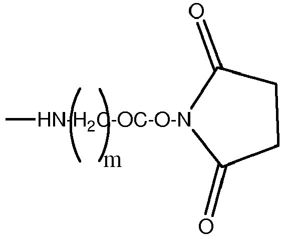

En d'autres termes, les dérivés de folate de l'invention sont donc caractérisés par la formule générale (I) telle que donnée ci-dessus, dans laquelle X est tel que défini précédemment et Y représente un groupement de couplage activé ou prêt à être activé pour permettre la formation d'une liaison amide, ester ou thioester, de préférence amide ou ester, avantageusement amide, entre ledit dérivé et un groupement fonctionnel porté par ladite molécule distinct M.

Selon un mode de réalisation particulièrement préféré, Y est un groupement de couplage activé ou prêt à être activé pour permettre la formation d'une liaison amide avec une amine primaire de ladite molécule M. Dans ce mode de réalisation, les molécules qui peuvent être couplées avec les dérivés de folate de l'invention sont toutes molécules qui possèdent naturellement une amine primaire, telles qu'une protéine, ou bien toutes molécules qui ont été modifiées chimiquement pour inclure une telle amine primaire, par exemple une biotine modifiée, présentant une amine primaire.

Par « groupement de couplage activé ou prêt à être activé », l'on entend un groupement fonctionnel adapté, le cas échéant après activation, pour permettre le couplage du dérivé de folate de l'invention avec le groupement fonctionnel porté par ladite molécule distincte M (par exemple avec une amine primaire portée par cette dernière).

Selon un mode de réalisation particulièrement préféré, Y est un groupement de couplage activé qui permet de former directement une liaison amide avec un groupement fonctionnel porté par la molécule M (par exemple une amine primaire), sans que ce groupement ait besoin d'être modifié au préalable. A titre d'exemple, l'on peut citer les groupements suivants :
N₃, 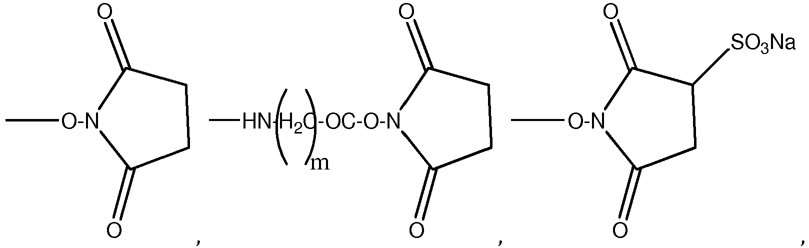 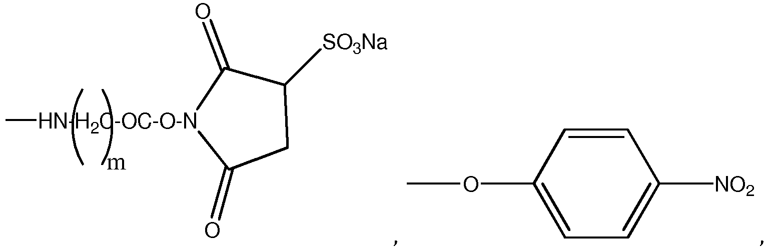 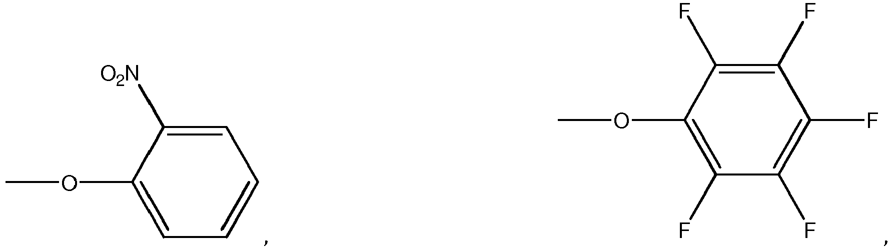 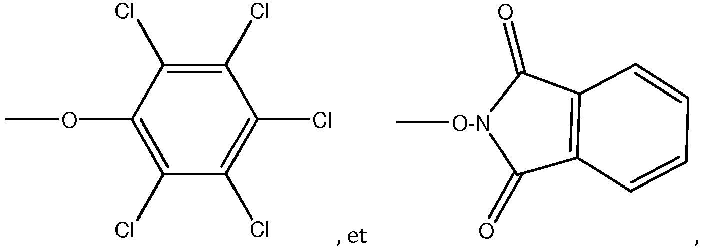
m étant un nombre entier, de préférence compris entre 1 et 10, ce qui constitue un mode de réalisation de l'invention.

Selon une alternative de la présente invention, Y est un groupement de couplage prêt à être activé, à savoir tout groupement qui doit être activé, par des méthodes connues de l'homme du métier, pour être capable de former une liaison amide, ester ou thioester, de préférence amide ou ester, avantageusement amide.

Selon un mode de réalisation particulièrement préféré, Y est un groupement de couplage prêt à être activé pour former une liaison amide entre le dérivé de folate selon l'invention et ladite molécule M. De tels groupements possèdent un groupe -OH ou -COOH. A titre d'exemples, l'on peut citer les groupements -OH et -NH-(CH₂)m-COOH ; m étant un nombre entier, de préférence compris entre 1 et 10, ce qui constitue un mode de réalisation de l'invention.

Selon un mode de réalisation particulier de l'invention, m est compris entre 1 et 5, ou encore entre 1 et 3.

Selon un autre mode de réalisation, Y est choisi parmi -OH, -NH-(CH₂)ₘ-COOH, et 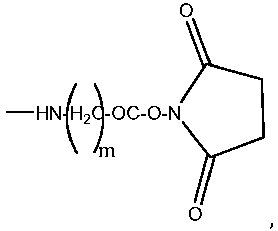 m étant compris entre 1 et 10 et de préférence étant égal à 1.

Selon un autre mode de réalisation particulièrement préféré, le dérivé de formule générale (I) est utilisé sous forme de conjugué avec ladite molécule M, et ledit conjugué étant représenté par la formule générale (III) suivante : 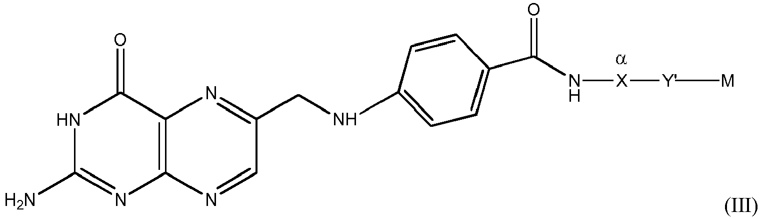
dans laquelle X et M sont tels que définis précédemment, et dans laquelle Y' est un dérivé du groupement fonctionnel Y après couplage du dérivé de formule générale (I) avec ladite molécule M,
ladite molécule M étant de préférence une molécule marqueur Mm.

Ce conjugué de formule générale (III) est différent du composé NSP-DMAE-HD-ptéroate représenté par la formule (A) supra..

De préférence, Y' est représenté par la formule générale (IV) suivante : 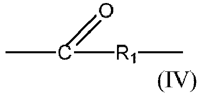 ou par la formule générale (V) suivante : 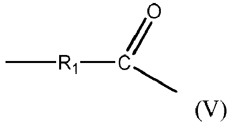
dans lesquelles R1 est -NH-, -O-, ou -S-, de préférence -NH- ou -O-, avantageusement -NH- ;
de préférence Y' étant représenté par la formule générale (IV).

Lorsque Y' correspond à la formule générale (IV), le conjugué selon l'invention peut être représenté par la formule générale (III') suivante : 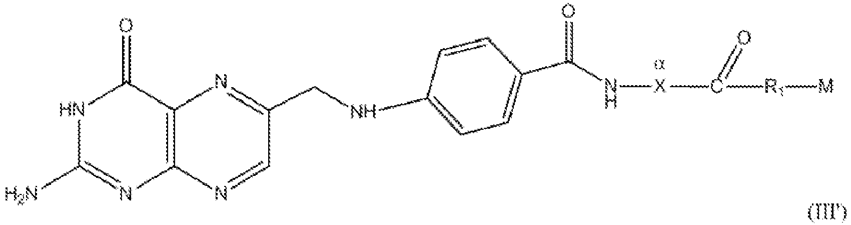 dans laquelle X, R₁ et M sont tels que définis précédemment.

Selon un mode de réalisation particulièrement préféré, R₁ est -NH- et la formule (III') est comme suit : 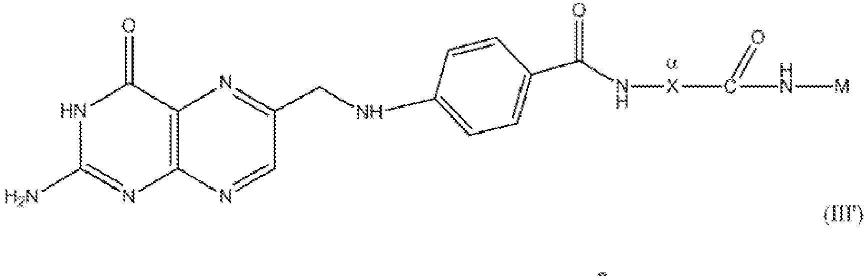 dans laquelle X et M sont tels que définis précédemment.

Selon un mode de réalisation particulièrement préféré, toujours lorsque Y' est un groupement de formule générale (IV), X est -(CH₂)ₙ- et le conjugué selon l'invention est représenté par la formule générale (III') suivante : 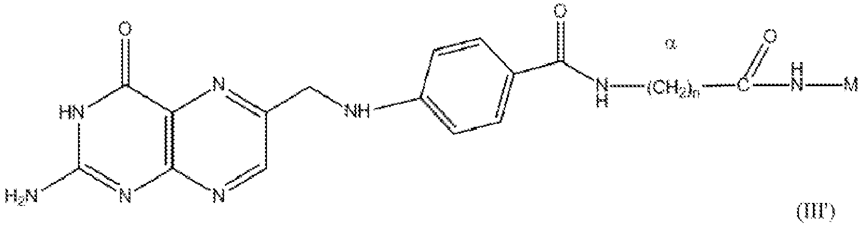 dans laquelle M et n sont tels que définis précédemment. Ainsi, n est un nombre entier compris entre 1 et 10. Selon un mode de réalisation préféré, n est un nombre entier compris entre 2 et 7, de préférence entre 3 et 5, avantageusement n est le nombre entier 3 ou 5.

Lorsque Y' correspond à un groupement de formule générale (V), le conjugué selon l'invention est représenté par la formule générale (III") : 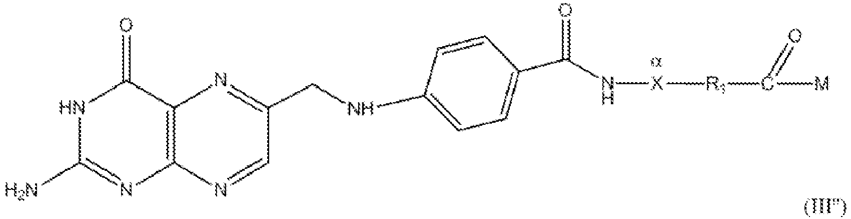 dans laquelle X, R₁ et M sont tels que définis précédemment.

Selon un mode de réalisation préféré, R₁ est -NH- et le conjugué selon l'invention répond alors à la formule (III") suivante : 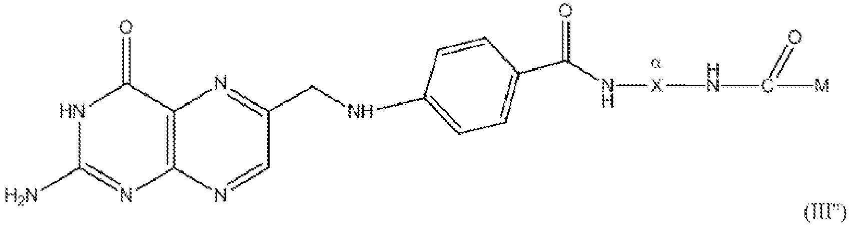 dans laquelle X et M sont tels que définis précédemment.

Selon un mode de réalisation particulièrement préféré, X est -(CH₂)ₙ- et le conjugué selon l'invention répond à la formule générale (III") suivante : 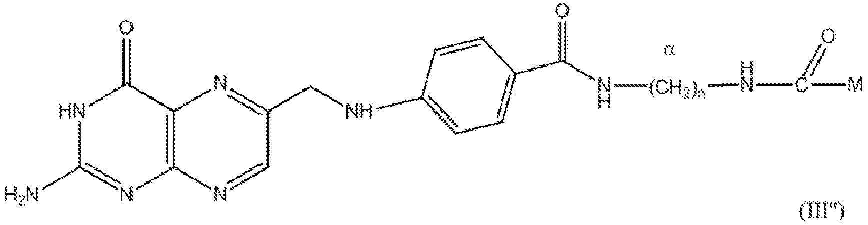 dans laquelle M et n sont tels que définis précédemment. Ainsi, n est un nombre entier compris entre 1 et 10. Selon un mode de réalisation préféré, n est un nombre entier compris entre 2 et 7, de préférence entre 3 et 5, avantageusement n est le nombre entier 3 ou 5.

Un autre objet de l'invention concerne un procédé de dosage *in vitro* du/des folate(s) dans un échantillon, tel qu'un échantillon biologique, ledit dosage étant un immunodosage par compétition non radio-isotopique, ledit procédé comprenant les étapes suivantes :
a) mettre en présence, au sein dudit échantillon biologique, (i) au moins un partenaire de liaison du/des folate(s), tel qu'un anticorps adapté pour se lier au(x) folate(s) ou tel que le récepteur des folates, avec (ii) au moins un composé choisi parmi un dérivé de folate tel que défini précédemment et un conjugué selon l'invention, au moins l'un desdits composés (i) et (ii) étant adapté pour émettre un signal,
b) éventuellement laisser un laps de temps suffisant pour permettre la réaction de compétition,
c) mesurer l'intensité du signal et en déduire la concentration en folate(s) présente dans ledit échantillon biologique par référence à une courbe de calibration établissant une relation entre intensité du signal mesurée et concentration en folate(s).

Ce procédé de dosage peut être mis en oeuvre dans un échantillon biologique d'origine clinique, à savoir prélevé à partir d'un patient humain ou animal. En outre, ce procédé de dosage trouve également à s'appliquer au dosage du/des folate(s) dans des échantillons d'origine agroalimentaire tels que des aliments destinés à l'alimentation humaine ou animale voire des compléments alimentaires. D'une manière générale, le procédé de dosage selon l'invention est applicable chaque fois qu'un dosage du ou des folate(s) est souhaité/nécessaire au sein d'un échantillon donné.

Quel que soit l'échantillon, d'origine clinique ou d'origine alimentaire, le dosage des folates nécessite une étape préalable de traitement de l'échantillon afin de dissocier les folates d'autres molécules présentes dans ces échantillons et avec lesquelles ils interagissent. De telles techniques de dissociations sont bien connues de l'homme du métier. A titre d'exemple, nous pouvons citer le brevet européen EP 0382334 qui divulgue une méthode de préparation des échantillons de sérum pour les dosages de la vitamine B12 et des folates. Le brevet US 4,418,151 divulgue une méthode de pré-traitement alternative. La notice du kit Abbott Axsym Folate (Cat. No. B7K460, Abbott Laboratories) décrit un protocole de préparation d'un hémolysat à partir d'un échantillon de sang total afin de rendre accessible au dosage toutes les molécules de folates présentes dans les érythrocytes.

L'homme du métier, très au fait des techniques d'immunodosage par compétition non radio-isotopique parviendra, sans difficulté excessive, à mettre en oeuvre les étapes b) et c). En particulier, il saura parfaitement comment construire une courbe de calibration, sur la base d'échantillons contenant des concentrations connues en folate(s), établissant ainsi une relation entre intensité du signal mesuré et concentration en folate(s).

Tel que mentionné précédemment, le dosage immunologique par compétition (également dénommé « immunodosage par compétition ») est un dosage largement connu de l'homme du métier. Il consiste à doser l'analyte, ici le folate, dans un échantillon d'intérêt, en créant une compétition entre l'analyte de l'échantillon et un dérivé de cet analyte, ici le dérivé de folate selon l'invention, vis-à-vis de la fixation à un partenaire de liaison d'origine immunologique (par exemple un anticorps ou un fragment d'anticorps) ou non (par exemple le récepteur des folates). La liaison du dérivé de l'analyte d'intérêt et du partenaire de liaison est mise en évidence grâce à la présence d'un traceur.

Le dérivé de l'analyte (en l'espèce le dérivé de folate) peut être utilisé dans la réaction de compétition, comme indiqué précédemment, sans couplage préalable ou après couplage à un marqueur pour former un conjugué ou traceur.

Lorsque le dérivé d'analyte (en l'espèce le dérivé de folate) n'est pas couplé à un marqueur (auquel cas, il ne constitue pas le traceur mais bien le partenaire de capture), le partenaire de liaison est alors marqué pour constituer le traceur de la réaction. Lorsque le dérivé d'analyte (en l'espèce le dérivé de folate) est couplé à un marqueur (en l'espèce à une molécule marqueur Mm) pour former un conjugué, ce dernier constitue alors le traceur et le partenaire de liaison devient alors le partenaire de capture.

Le signal mesuré, émis par le traceur, est alors inversement proportionnel à la quantité de folate(s) présent(s) dans l'échantillon à doser.

En tant que partenaire de liaison du/des folate(s), l'on utilise toute molécule capable de se lier au(x) folate(s). A titre d'exemples de partenaire de liaison au(x) folate(s), l'on peut citer les anticorps, les fragments d'anticorps, les nanofitines, le récepteur des folates ou toute autre protéine connue pour se lier au(x) folate(s) avec une affinité suffisante pour réaliser l'immunodosage par compétition non radio-isotopique selon l'invention.

Lorsque l'on utilise, en tant que partenaires de liaison, des anticorps, ces derniers peuvent être, par exemple, des anticorps polyclonaux ou monoclonaux.

Les anticorps polyclonaux peuvent être obtenus par immunisation d'un animal avec, comme immunogène, le folate cible, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixé un antigène spécifiquement reconnu par les anticorps, notamment l'immunogène.

Les anticorps monoclonaux peuvent être obtenus par la technique des hybridomes largement connue de l'homme du métier. Les anticorps monoclonaux peuvent être également des anticorps recombinants obtenus par génie génétique, par des techniques bien connues de l'homme du métier.

A titre d'exemples de fragments d'anticorps, on peut citer les fragments Fab, Fab', F(ab')₂ ainsi que les chaînes scFv (Single chain variable fragment), dsFv (Double-stranded variable fragment). Ces fragments fonctionnels peuvent notamment être obtenus par génie génétique.

Les nanofitines (nom commercial) sont de petites protéines qui, comme les anticorps, sont capables de se lier à une cible biologique permettant ainsi de la détecter, de la capturer ou tout simplement de la cibler au sein d'un organisme.

Les partenaires de liaison utilisés peuvent être spécifiques ou non du/des folate(s). Ils sont dits spécifiques quand ils sont capables de se lier de façon exclusive ou quasi exclusive au(x) folate(s). Ils sont dits non spécifiques lorsque la sélectivité de liaison au(x) folate(s) est faible et qu'ils sont capables de se lier à d'autres ligands, tels que des protéines ou anticorps. Selon un mode de réalisation préféré, l'on utilise, dans le cadre du procédé d'immunodosage non radio-isotopique par compétition selon la présente invention, au moins un partenaire de liaison spécifique.

Les partenaires de liaison ou les dérivés de folate selon l'invention, lorsqu'ils sont utilisés en capture, peuvent être liés ou non à un support par toute technique connue de l'homme du métier.

La deuxième étape b) du procédé de l'invention est une étape classique d'un procédé d'immunodosage par compétition.

La dernière étape c) du procédé selon l'invention consiste à déterminer la concentration en folate(s). Le signal mesuré est inversement proportionnel à la quantité de folate(s) dans l'échantillon. Afin de déterminer la concentration en folate(s), l'intensité du signal est mesurée et cette intensité est reportée sur une courbe de calibration obtenue au préalable par des techniques largement connues de l'homme du métier. Ainsi, par exemple, la courbe de calibration est obtenue en effectuant un immunodosage par compétition en utilisant le même partenaire de liaison ainsi que des quantités croissantes de folate(s). Une courbe est ainsi obtenue en mettant, par exemple, en abscisse la concentration en folate(s) et en ordonnée le signal correspondant obtenu après immunodosage.

Lorsque le composé (ii) est un conjugué selon l'invention, tel que décrit précédemment, ce qui constitue un mode de réalisation de l'invention, le signal est généré par le marquage de la molécule marqueur Mm, tel que décrit précédemment.

Lorsque le composé (ii) est un dérivé de folate de l'invention, tel que décrit précédemment, c'est-à-dire qu'il n'est pas lié à une molécule marqueur Mm, le signal consiste en la lecture directe de la liaison partenaire de liaison/dérivé de folate, qui peut notamment être réalisée par résonance plasmonique de surface ou par voltamétrie cyclique.

De préférence, le composé (ii) est un conjugué selon l'invention.

Tel qu'indiqué précédemment, les dérivés et conjugués de folate selon l'invention peuvent être utilisés pour doser un/des folates au sein d'un échantillon. De préférence, ces dérivés et conjugués sont utilisés pour doser une pluralité (au moins deux) folates, à savoir plusieurs formes folates (notamment les formes réduites) au sein dudit échantillon.

Selon un mode de réalisation particulier de l'invention, les dérivés et conjugués de folate susvisés sont utilisés pour doser le folate total dans un échantillon.

L'invention concerne également une méthode de diagnostic *in vitro* visant à déterminer si un patient humain ou animal, de préférence humain, présente une carence en folate(s), ladite méthode comprenant les étapes suivantes :
a) au sein d'un échantillon biologique, de préférence de sang total, de plasma ou de sérum, prélevé à partir dudit patient, doser le/les folate(s) en mettant en oeuvre le procédé de dosage *in vitro* selon l'invention, afin d'en déduire la concentration en folate(s) dans l'échantillon biologique,
b) comparer ladite concentration avec une valeur seuil, correspondant à une concentration prédéterminée en folate(s) au-dessous de laquelle un patient est considéré comme carencé en folate(s), et
c) si la concentration dosée est inférieure à ladite valeur seuil, en déduire que le patient présente une carence en folate(s).

Un autre objet de l'invention concerne un dérivé de folate de formule générale (I), adapté pour être utilisé dans un immunodosage par compétition non radio-isotopique : 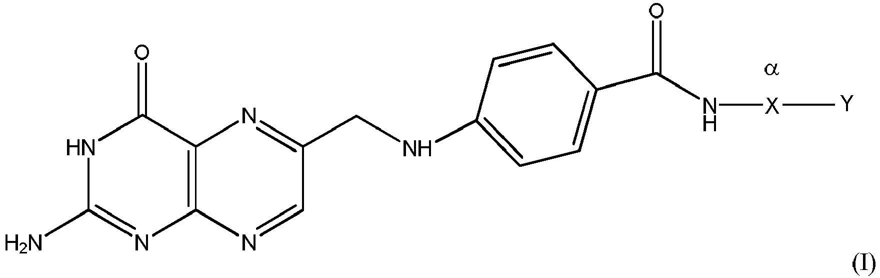 dans laquelle :
- X est telle que définie précédemment;
- Y est un groupement de type centre électrophile ou de type centre nucléophile, de préférence un groupement de type centre électrophile, adapté pour permettre la formation d'une liaison amide, ester, ou thioester, de préférence amide ou ester, avantageusement amide, entre Y et un groupement fonctionnel porté par une molécule distincte M ; et dans laquelle :
   - lorsque X est une chaîne hydrocarbonée linéaire et saturée comprenant un nombre d'atomes de carbone égal à 2, Y n'est pas un groupement -(O-CH₂-CH₂)₂-NH₂ ;
   - lorsque Y est un groupement de type centre nucléophile consistant en une amine primaire, X comprend un nombre d'atomes de carbone différent de 6,
   - lorsque Y est un groupement de type centre électrophile, ce dernier répond à la formule générale (II) suivante : 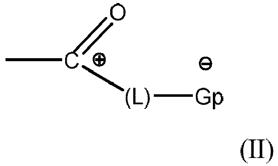
dans laquelle Gp est un groupement partant, éventuellement relié à la fonction carbonyle par un bras L, Gp étant adapté pour être dissocié du groupement de type centre électrophile lors d'une réaction avec un groupement nucléophile porté par ladite molécule distincte M, tel qu'une amine primaire, et dans lequel, lorsque L est absent et Gp est -OH, X comprend un nombre d'atomes de carbone supérieur à 4.

Selon un mode de réalisation particulier, le groupement fonctionnel Y ne comprend pas de motif -(O-CH₂-CH₂)-.

Ce dérivé de folate de formule générale (I) est adapté à la mise en oeuvre d'un immunodosage par compétition non radio-isotopique du/des folates dans un échantillon. Il n'inclut donc pas de radio-isotope/radio-élément.

Le susdit dérivé de folate de formule générale (I) en tant que tel est non-marqué. Le cas échéant, ledit dérivé de folate sera marqué après couplage avec une molécule marqueur Mm, ledit couplage comprenant la formation d'une liaison covalente entre le groupement fonctionnel Y dudit dérivé de folate et un groupement fonctionnel porté par ladite molécule marqueur Mm.

Le dérivé de folate de formule générale (I) est différent du composé NSP-DMAE-HD-ptéroate représenté par la formule (A) supra.

Selon un mode de réalisation particulier, Y est différent du groupement (C) :

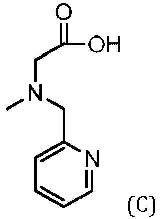

Selon un autre mode de réalisation particulier, lorsque X est une chaîne hydrocarbonée linéaire et saturée comprenant un nombre d'atomes de carbone égal à 2, Y est différent des groupements (D) et (E) suivants :

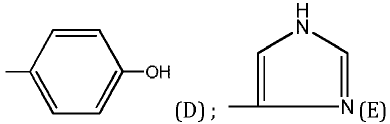

Selon une variante de cet « autre mode de réalisation particulier », Y est différent des groupements (D) et (E) susvisés, et ce quelle que soit la définition de X.

De préférence, ladite réaction du groupement de type centre électrophile de formule générale (II) avec ledit groupement nucléophile porté par la dite molécule M - tel qu'une amine primaire - est une réaction de substitution nucléophile.

Selon un mode de réalisation particulier, lorsque Y est un groupement de type centre nucléophile, ce dernier consiste en un groupement fonctionnel différent d'une amine primaire.

Selon un mode de réalisation préféré, lorsque Y est un groupement de type centre électrophile répondant à la formule générale (II), (L)-Gp est un groupement choisi parmi : -OH, -NH-(CH₂)ₘ-COOH, -N₃, 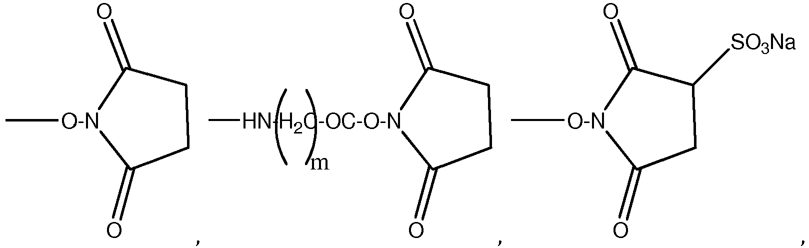 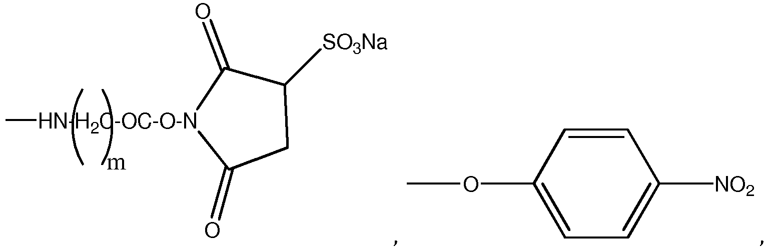 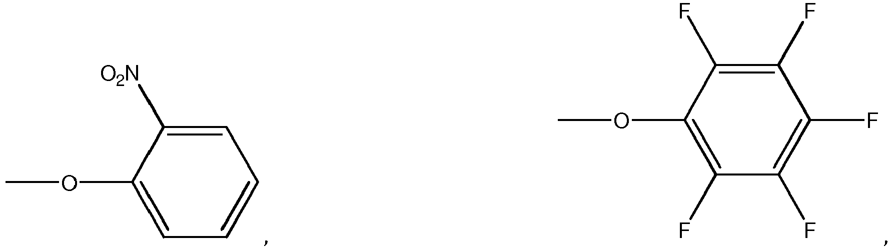 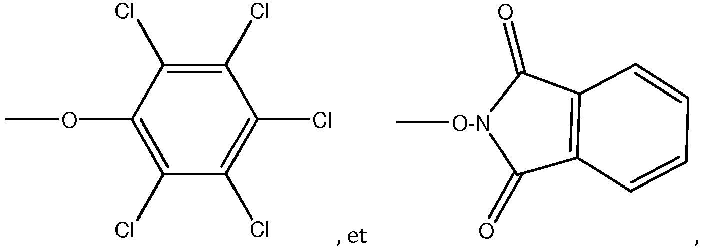 m étant un nombre entier compris entre 1 et 10, de préférence compris entre 1 et 5, avantageusement compris entre 1 et 3, dans lequel lorsque L est absent et Gp est -OH, X comprend un nombre d'atomes de carbone supérieur à 4.

De préférence, lorsque Y est un groupement de type centre électrophile de formule (II), (L)-Gp est choisi parmi :
-OH et -NH-(CH₂)ₘ-COOH et 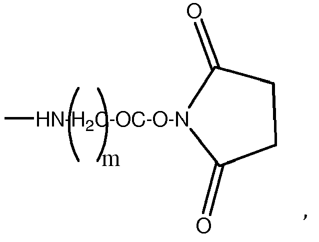
m étant compris entre 1 et 10 et de préférence m étant égal à 1,
dans lequel lorsque L est absent et Gp est -OH, X comprend un nombre d'atomes de carbone supérieur à 4.

Selon un mode de réalisation particulièrement avantageux, lorsque Y est un groupement de type centre électrophile de formule (II), (L)-Gp est :

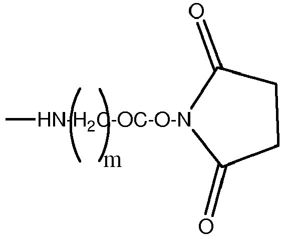

Selon un mode de réalisation particulier, et toujours lorsque Y est un groupement de type centre électrophile de formule générale (II), Gp est un groupement différent d'un groupement -OH (groupement hydroxyle).

L'invention concerne également un conjugué comprenant un dérivé de folate selon l'invention et une molécule distincte M, ledit dérivé et ladite molécule M étant liés par au moins une liaison amide, ester, ou thioester, de préférence une liaison amide ou ester, avantageusement par une liaison amide, entre Y et un groupement fonctionnel porté par ladite molécule M.

Selon un mode de réalisation particulièrement préféré, ladite molécule M est choisie parmi les bras chimiques ou « linkers » ou parmi les molécules marqueurs Mₘ, ladite molécule M étant de préférence une molécule marqueur Mm permettant le marquage direct ou indirect dudit dérivé de folate (ce dernier étant alors dénommé « conjugué marqué »).

Un autre objet de l'invention concerne un kit permettant la mise en oeuvre du procédé selon l'invention, ledit kit comprenant :
- (i) au moins un partenaire de liaison du ou des folate(s), tel qu'un anticorps adapté pour se lier au(x) folate(s) ou tel que le récepteur des folates,
- (ii) au moins un composé choisi parmi un dérivé de folate tel que défini précédemment et un conjugué selon l'invention,
   au moins l'un desdits composés (i) et (ii) étant adapté pour émettre un signal,
et
- au moins un moyen de calibration.

Bien entendu, ce kit (également dénommé « trousse de détection ») peut comprendre d'autres constituants permettant ou favorisant la mise en oeuvre de l'immunodosage selon l'invention, tels que, par exemple, des tampons de lavage et un ou plusieurs autre(s) réactif(s) permettant la visualisation du marquage ou l'émission d'un signal détectable.

Les dérivés de folate de l'invention peuvent être utilisés de deux manières différentes dans les procédés de dosage du ou des folate(s) par immunodosage par compétition, tels que les tests diagnostiques. En effet, soit ils sont utilisés tels quels, soit ils sont utilisés couplés à une autre molécule pour former un conjugué.

Ladite « autre molécule » est soit un marqueur direct ou indirect, soit un bras chimique ou « linker », soit un composé chimique dont le couplage avec un dérivé de folate présente un intérêt, notamment pour la mise en oeuvre d'un dosage du folate par immunodosage par compétition (de préférence non radio-isotopique).

Ainsi, la présente invention a également pour objet les conjugués comprenant ou constitués d'un dérivé de folate tels que décrits précédemment et d'une autre molécule, en particulier d'un marqueur ou d'un bras chimique.

Par marqueur, l'on entend toute molécule capable de générer directement ou indirectement un signal détectable. Une liste non limitative de ces marqueurs de détection directe consiste en :
- les enzymes qui produisent un signal détectable par exemple par colorimétrie, fluorescence, luminescence, comme la peroxydase de raifort, la phosphatase alcaline, la β-galactosidase, la glucose-6-phosphate déshydrogénase,
- les chromophores comme les composés fluorescents, luminescents, colorants,
- les molécules fluorescentes telles que les Alexa ou les phycocyanines,
- les sels électrochimiluminescentes tels que des dérivés organo-métalliques à base d'acridinium ou de ruthénium.

Des systèmes indirects de détection peuvent également être utilisés, comme par exemple des ligands capables de réagir avec un anti-ligand. Le ligand correspond alors à la molécule marqueur Mm pour constituer, avec le dérivé de folate mentionné supra, le conjugué de l'invention.

Les couples ligand/anti-ligand sont bien connus de l'homme du métier. A titre d'exemple, l'on peut notamment citer les couples suivants : biotine/streptavidine, haptène/anticorps, antigène/anticorps, peptide/anticorps, sucre/lectine, polynucléotide/complémentaire du polynucléotide.

L'anti-ligand peut alors être détectable directement par les marqueurs de détection directe décrits précédemment ou être lui-même détectable par un autre couple ligand/anti-ligand, et ainsi de suite.

Ces systèmes indirects de détection peuvent conduire, dans certaines conditions, à une amplification du signal. Cette technique d'amplification du signal est bien connue de l'homme du métier, et l'on pourra notamment se reporter aux demandes de brevet antérieures FR98/10084 et WO-A-95/08000 de la Demanderesse.

Selon le type de marquage utilisé, l'homme du métier ajoutera des réactifs permettant la visualisation du marquage ou l'émission d'un signal détectable par tout type d'appareil de mesure approprié, comme par exemple un spectrophotomètre, un spectrofluorimètre ou encore une caméra (par exemple une caméra haute définition). Par bras chimique ou « linker », l'on entend toute molécule pouvant être couplée avec le dérivé selon la présente invention, ladite molécule étant en outre capable de fixation sur une phase solide, de manière covalente ou non-covalente, de manière sélective ou non-sélective.

Tel qu'indiqué précédemment, les dérivés de folate et les conjugués correspondants selon la présente invention sont particulièrement utiles pour la détermination *in vitro* de la concentration en folate(s) dans un échantillon d'origine clinique (par exemple un échantillon biologique prélevé à partir d'un patient humain ou animal) ou d'un échantillon d'origine agroalimentaire (prélevé à partir d'un aliment ou d'un complément alimentaire).

La détermination de la concentration en folate(s) en utilisant le dérivé ou le conjugué selon la présente invention pourra être réalisée dans le surnageant de culture ou encore dans le lysat cellulaire.

La description décrit également un procédé d'obtention d'un dérivé selon l'invention, ledit procédé comprenant les étapes suivantes :
a) synthétiser, à partir de l'acide ptéroïque, le composé de formule générale (VI) : 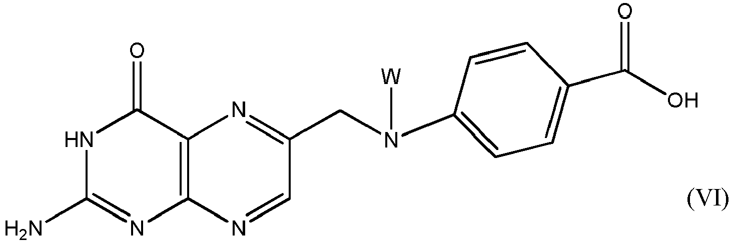 dans laquelle W représente un groupement augmentant la solubilité dudit composé en solvant(s) organique(s), tel qu'un groupement trifluoroacétyl (COCF3),
b) mettre à réagir ledit composé de formule (VI), obtenu à l'étape a), avec un composé de formule générale (VII) suivante :

   NH₂-X-Y"-Z (VII)

   dans des conditions permettant d'obtenir, par couplage, le composé de formule générale (VIII) suivante : 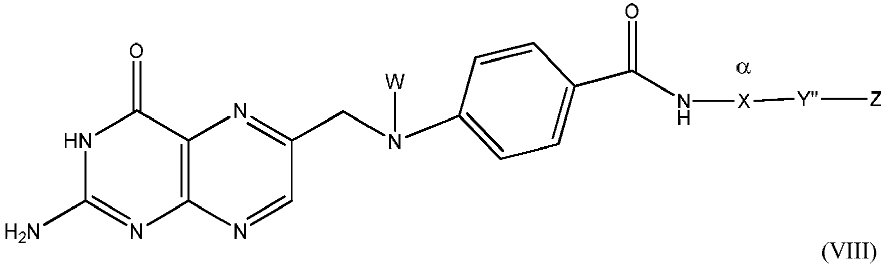
   X étant tel que défini ci-dessus,
   Z étant un groupement inerte, de préférence alkyle ou aryle, et
   Y" étant un dérivé du groupement Y (tel que défini supra) après liaison avec ledit groupement Z.
c) déprotéger, dans des conditions permettant cette déprotection, le composé de formule (VIII), afin d'obtenir le dérivé de folate de formule (I) selon l'invention.

Selon un mode de réalisation préféré, l'on met à réagir, à l'étape b), ledit composé de formule (VI), obtenu à l'étape a), avec un composé de formule générale (VII) suivante :

NH₂-X-Y"-Z (VII)

dans lequel Y" est -C(O)O-,
afin de préparer un dérivé de folate de formule (I), dans lequel Y est -COOH.

Dans ce mode de réalisation préféré, si Z est un groupement méthyle ou éthyle, Y-Z est respectivement -C(O)O-CH3 ou -C(O)O-CH2-CH3 dans les composés de formules (VII) et (VIII). Si Z est un groupement aryle, alors Y-Z est -C(O)O-Ar au sein desdits composés (Ar symbolisant ce groupement aryle).

A l'issue de l'étape de déprotection c), et toujours dans ce mode de réalisation préféré, le susdit groupement -C(O)O- résulte en un groupement -COOH. Le dérivé de folate de formule générale (I) selon l'invention ainsi obtenu est dénommé dérivé de folate « acide ». Selon un aspect préféré de ce mode de réalisation, le procédé comprend alors, après l'étape c), l'étape additionnelle suivante :
d) obtenir, à partir dudit dérivé de folate acide (dans lequel Y = -COOH), l'ester du dérivé de folate-NHS, à savoir le composé répondant à la formule générale (IX) suivante : 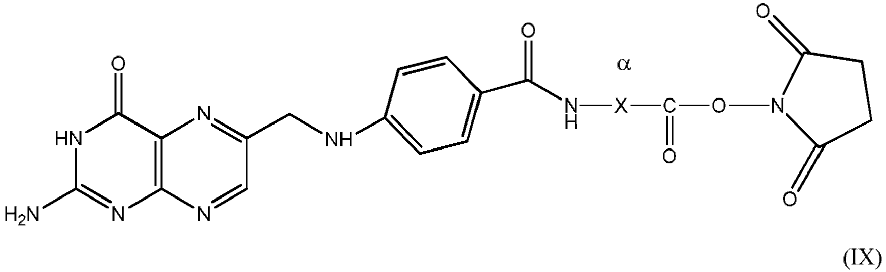 dans laquelle X est tel que défini précédemment.

Ce composé de formule générale (IX) représente un des dérivés de folate préférés, voire le dérivé de folate préféré au sens de la présente invention. Avantageusement, X est une chaîne hydrocarbonée aliphatique, linéaire et saturée comprenant un nombre d'atomes de carbones compris entre 1 et 10, de préférence entre 2 et 7, avantageusement entre 3 et 5.

Les méthodes d'obtention des esters NHS (N-hydroxysuccinimides) sont bien connues de l'homme du métier. Des exemples d'obtention des dérivés de folate de formule (IX) selon l'invention sont présentés ci-après (cf. exemples 3 et 4 infra), à titre illustratif et non limitatif.

L'acide ptéroïque est un composé disponible dans le commerce. Malheureusement, ce composé s'avère peu soluble dans les solvants organiques courants. Ce dernier est donc transformé, à l'étape a), en un composé de formule générale (VI) pour faciliter la suite du présent procédé. De préférence, ce composé de formule (VI) est l'acide N-trifluoroacétylptéroïque (W = -COCF3). Dans ce cas, l'acide ptéroïque est mis à réagir, de préférence, avec de l'anhydride trifluoroacétique, avantageusement sous protection d'azote.

Tel qu'indiqué précédemment, l'étape b) est réalisée des conditions réactionnelles permettant d'obtenir la réaction souhaitée. Ces conditions réactionnelles incluent, de préférence, l'utilisation d'un agent de couplage.

Tel qu'indiqué précédemment, le groupement inerte Z consiste, par exemple, en un groupement méthyle ou éthyle (notamment lorsque Y" est -C(O)O-, pour donner-C(O)O-Me ou -C(O)O-Et) ou en un groupement Fmoc (notamment lorsque Y" est -NH-, pour donner -NH-Fmoc).

Selon un mode de réalisation particulier, l'étape b) comprend les deux sous-étapes suivantes :
b.1) former, à partir du composé de formule (VI), obtenu à l'étape a), un intermédiaire actif tel que l'isobutylformiate d'acide N-trifluoroacétylptéroïque de formule générale (VI') suivante : 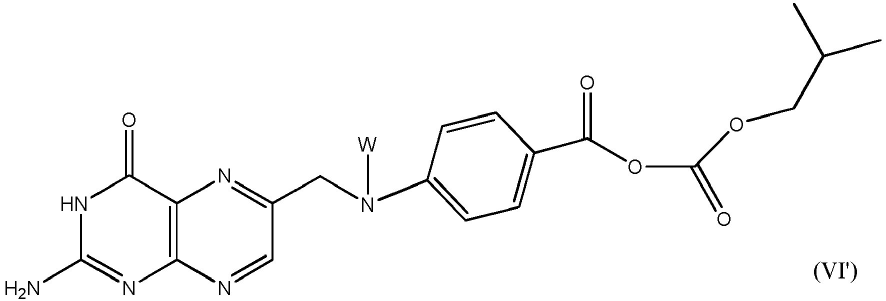
b.2) mettre à réagir ledit un intermédiaire actif obtenu à l'étape b.1), avec un composé de formule générale (VII) suivante :

   NH₂-X-Y"-Z (VII)

   dans des conditions permettant d'obtenir, par couplage, le composé de formule générale (VIII) suivante : 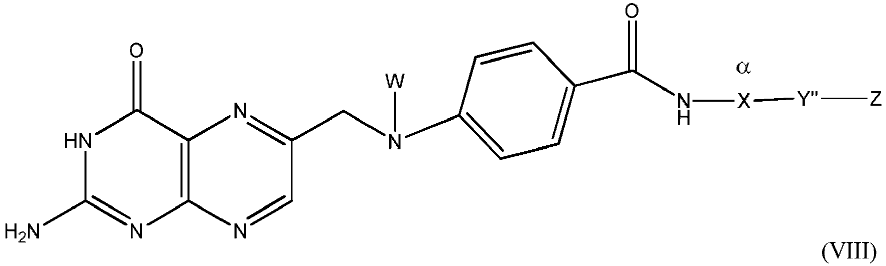 dans laquelle W, X, Y" et Z sont tels que définis précédemment.

Selon un mode de réalisation préféré, lors des étapes b) et/ou b.2) susvisées, le couplage est obtenu par transamidification.

L'étape de déprotection c) résulte en l'élimination du groupement W et, de manière concomitante ou subséquente, du groupement Z, avantageusement sous protection d'azote.

Cette étape de déprotection c) est, de préférence, effectuée en milieu basique, par exemple dans une solution de NaOH. Cette solution basique permet l'élimination du groupement W et, de manière concomitante, du groupement Z (consistant par exemple en un groupement méthyle ou éthyle), par réaction de saponification.

### Description détaillée

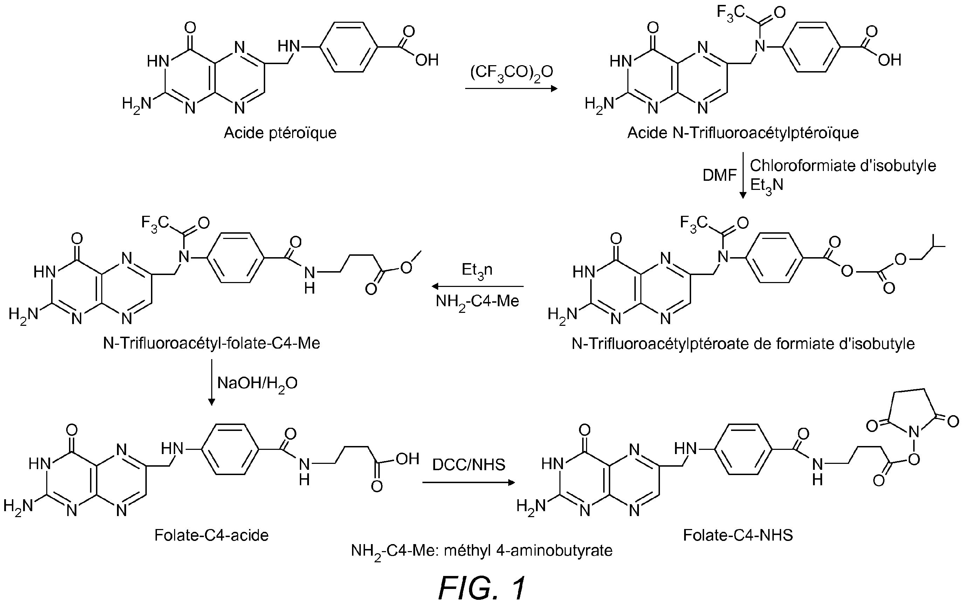

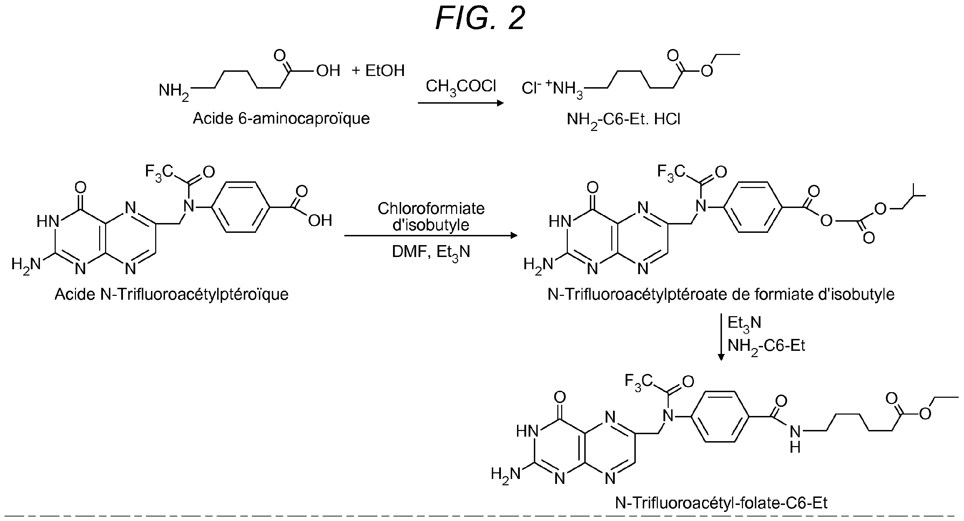

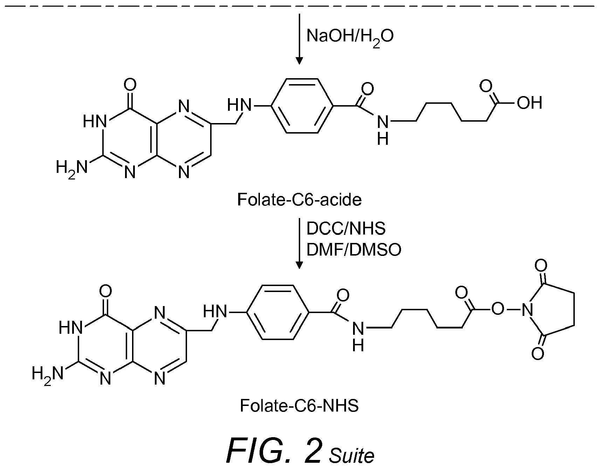

L'invention sera mieux comprise à l'aide des exemples suivants qui sont donnés à titre illustratif et non limitatif, en référence aux figures 1 et 2, dans lesquelles :
- la figure 1 présente les étapes de synthèse du folate-C4-acide (acide 4-(4-((2-amino-4-oxo-3,4-dihydropteridin-6-yl)méthylamino)benzamido) butanoïque) et du folate-C4-NHS, et
- la figure 2 présente les étapes de synthèse du folate-C6-acide (acide 6-(4-((2-amino-4-oxo-3,4-dihydropteridin-6-yl)méthylamino)benzamido) hexanoïque) et du folate-C6-NHS.

### Exemple 1 : Préparation de l'acide 4-(4-((2-amino-4-oxo-3,4-dihydropteridin-6-yl)méthylamino)benzamido)butanoïque(folate-C4-acide)

A des fins de clarté, le composé dénommé « folate-C4-acide » est un dérivé selon l'invention, répondant à la formule générale (I) suivante : 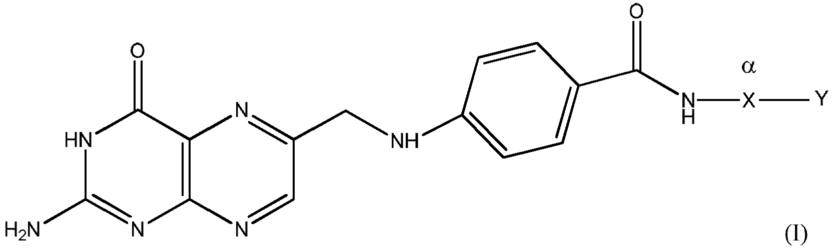 dans laquelle X est une chaîne hydrocarbonée aliphatique, linéaire et saturée, comprenant 3 atomes de carbone, et Y est un groupement de type centre électrophile répondant à la formule générale (II) : 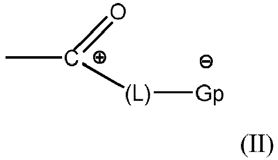 dans laquelle L est absent et Gp est -OH.

### 1.1. Introduction

Les réactifs acide ptéroïque (CAS-Nr. 119-24-4), anhydride trifluoroacétique (CAS-Nr. 407-25-0), méthyl 4-aminobutyrate (CAS-Nr. 3251-07-8), chloroformiate d'isobutyle (CAS-Nr. 543-27-1), acide amino-6-hexanoïque (CAS-Nr. 60-32-2 appelé aussi acide 6-aminocaproïque) et dichlorométhane anhydre (CAS-Nr. 75-09-2) ont été obtenus chez Sigma-Aldrich.

A chaque étape de synthèse, la chromatographie liquide haute pression (HPLC) est utilisée pour le suivi d'avancement de réaction et l'analyse des produits. La colonne utilisée est une Vydac 218TP54, C18, 250x4.6mm, 5µm et l'éluant est un mélange acétonitrile, eau (0,1% acide trifluoroacétique) en gradient.

### 1.2. Etape 1 : obtention de l'acide N-trifluoroacétylptéroïque

Dans un ballon de 50 mL muni d'une agitation magnétique, d'une entrée et d'une sortie de l'azote de protection, 500 mg (1,60 mmole) d'acide ptéroïque sont introduits. 10mL d'anhydride trifluoroacétique sont ajoutés sous protection de l'azote, goutte à goutte, pendant 30 minutes. Le mélange de réaction est agité à température ambiante pendant 24 heures à l'abri de lumière. Le milieu de réaction est évaporé sous pression réduite à température ambiante et le résidu est séché sous vide pendant 1 heure. Le produit obtenu est lavé avec 5mL de l'éther éthylique puis séché sous vide. Le produit est analysé en HPLC et directement utilisé pour la suite de la synthèse.

### 1.3. Etape 2 : obtention du N-trifluoroacétyl-folate-C4-Me

Un mélange de 171 mg (0,42 mmole) de l'acide N-trifluoroacétylptéroïque, 0,111 mL de triéthylamine (CAS-Nr. 121-44-8) et 2 mL de diméthlyformamide sec (DMF, CAS-Nr. 68-12-2) est préparé et agité à température ambiante sous l'azote pendant 45 minutes pour donner le milieu n°1. Dans un autre ballon de 10 mL, 150 mg (0,98 mmole) de méthyl 4-aminobutyrate hydrochloride sont mélangés avec 2 mL de DMF sec, puis 0,111 mL de triéthylamine sont ajoutés. Après une agitation pendant 30 secondes, le mélange obtenu est ajouté sous protection de l'azote dans le milieu n°1 préparé. L'agitation est maintenue à température ambiante pendant 3 heures et la réaction est contrôlée en HPLC. Le milieu de réaction est séché sans chauffer sous vide. Le résidu est purifié en chromatographie sur gel de silice 60 (0,040-0,063 mm, Merck Cat. No. 109385) avec l'éluant dichlorométhane/méthanol, 5/1, v/v. 85 mg de produit sont obtenus, ce qui correspond à un rendement de 40%. La pureté est de 96%, déterminée par HPLC.

### 1.4. Etape 3 : obtention du folate-C4-acide

85 mg (0,168 mmole) de l'acide N-trifluoroacétyl-folate-C4-Me préparé sont mélangés avec 6 mL de méthanol. 2 mL d'une solution de NaOH 1 N sont ajoutés. Le mélange est agité à température ambiante et à l'abri de lumière pendant 16 heures. Le milieu de réaction est neutralisé à pH 2 avec 50% d'acide trifluoroacétique puis séché sous pression réduite à température ambiante. Le résidu est lavé avec 10 mL d'eau déminéralisée et séché sous vide. 66 mg de folate-C4-acide sont obtenus, ce qui correspond à un rendement de 98%. La pureté est de 97,2%, déterminée par HPLC.

La synthèse du folate-C4-acide est résumée en figure 1.

### Exemple 2 : Préparation de l'acide 6-(4-((2-amino-4-oxo-3,4-dihydropteridin-6-yl)méthylamino)benzamido)hexanoïque(folate-C6-acide)

Le dérivé de folate dénommé « folate-C6-acide » est un dérivé selon la présente invention répondant à la formule générale (I) suivante : 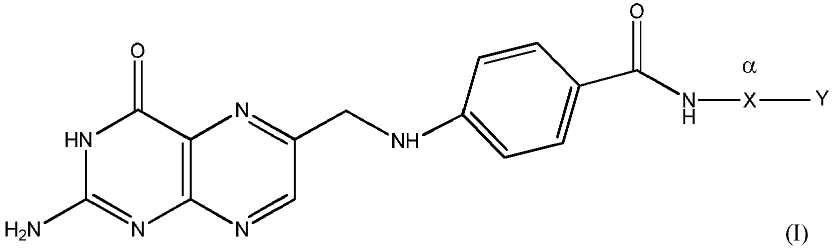 dans laquelle X est une chaîne hydrocarbonée aliphatique, linéaire et saturée, comprenant 5 atomes de carbone, et dans laquelle, Y est un groupement de type centre électrophile, répondant à la formule générale (II) suivante : 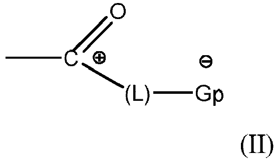 dans laquelle L est absent et Gp est -OH.

Le folate-C6-acide (acide 6-(4-((2-amino-4-oxo-3,4-dihydropteridin-6-yl)méthyl-amino)benzamido)hexanoïque) est synthétisé de façon analogue au folate-C4-acide. Dans l'étape 2 susvisée, le bras méthyl 4-aminobutyrate (NH₂-C4-Me) est remplacé par un bras éthyl 6-aminocaproate (NH₂-C6-Et, ester éthylique de l'acide amino 6-hexanoïque). Ce composé est préparé à partir de l'acide amino-6-hexanoïque en présence d'éthanol et de chlorure d'acétyle.

60 mg de folate-C6-acide sont obtenus à partir de 171 mg de l'acide N-trifluoroacétylptéroïque, ce qui correspond à un rendement global de 34%. La pureté est de 96%, déterminée par HPLC.

La synthèse du folate-C6-acide est résumée en Figure 2.

D'une manière générale, l'on pourra faire varier la longueur de la chaîne hydrocarbonée X dans le dérivé de formule générale (I) en utilisant, à l'étape 2, des réactifs aminoalcanoate d'alkyle dont la longueur de la partie hydrocarbonée de l'alcanoate varie (4 atomes de carbone pour obtenir le composé dénommé « folate-C4-acide » ; 6 atomes de carbone pour obtenir le composé dénommé « folate-C6-acide »).

### Exemple 3 : Préparation des esters de folate-C4-NHS

Les réactifs, N-hydroxysuccinimide (NHS, CAS-Nr. 6066-82-6), 1,3-dicyclohexylcarbodiimide (DCC, CAS-Nr. 538-75-0), diméthylsulfoxyde (DMSO, CAS-Nr. 67-68-5) et tétrahydrofurane (CAS-Nr. 109-99-9) ont été obtenus chez Sigma-Aldrich.

66 mg (0,166 mmole) de folate-C4-acide obtenus dans l'exemple 1, 28,8 mg (soit 1,5 x 0,166 mmole) de NHS et 4 mL de DMSO sec sont introduits dans un ballon. 41,1 mg (soit 1,2 x0,166 mmole) de DCC sont introduits après 2 minutes d'agitation. L'agitation est maintenue à température ambiante à l'abri de lumière pendant 48 heures. La HPLC est utilisée pour contrôler l'avancement de l'activation. 7 mg de NHS et 4 mg de DCC sont ajoutés et l'agitation est maintenue pendant 72 heures supplémentaires.

Le mélange réactionnel est filtré et la solution obtenue est mélangée avec 5 mL de tétrahydrofurane sec, puis 170 mL de dichlorométhane sec sont ajoutés. Le mélange est centrifugé après 15 minutes sans agitation pour récupérer le précipité qui est ensuite séché sous pression réduite, sans chauffer et à l'abri de la lumière. 42 mg de folate-C4-NHS sont obtenus, ce qui correspond à un rendement de 51%. La pureté est de 69%, déterminée par HPLC.

L'obtention de l'ester de folate-C4-NHS est représentée sur la figure 1 (cf. dernière étape réactionnelle).

### Exemple 4 : Préparation des esters de folate-C6-NHS

Comme pour l'ester de folate-C4-NHS, les réactifs utilisés sont les suivants : N-hydroxysuccinimide (NHS, CAS-Nr. 6066-82-6), 1,3-dicyclohexylcarbodiimide (DCC, CAS-Nr. 538-75-0), diméthylsulfoxyde (DMSO, CAS-Nr. 67-68-5) et tétrahydrofurane (CAS-Nr. 109-99-9), et ont été obtenus chez Sigma-Aldrich.

60 mg (0,14 mmole) de folate-C6 acide obtenus dans l'exemple 2, un mélange de solvants secs DMF (2,5 mL) et DMSO (3 mL) et 17,7 mg (1,1 x 0,14 mmole) de NHS sont introduits dans un ballon. 32 mg (1,1 x 0,14 mmole) de DCC sont ajoutés après 2 minutes d'agitation. L'agitation est maintenue à température ambiante, à l'abri de lumière, pendant 24 heures. La HPLC est utilisée pour contrôler l'avancement de l'activation. 30 mg de NHS et 30 mg de DCC sont ajoutés et l'agitation est maintenue pendant 96 heures supplémentaires.

Le milieu réactionnel est centrifugé pendant 3 minutes à 3000 tours/min et le liquide récupéré est mélangé avec 30 mL du mélange des solvants dichlorométhane/éther de pétrole (1/1) puis centrifugé à nouveau pour obtenir un précipité jaune. Le produit est séché sous pression réduite, sans chauffer et à l'abri de la lumière. 21 mg (rendement 29%) de folate-C6-NHS sont obtenus avec une pureté HPLC de 83,1%.

La synthèse de l'ester de folate-C6-NHS est résumée sur la figure 2 (cf. dernière étape réactionnelle).

### Exemple 5 : Préparation des conjugués folate-C4-NHS-phosphatase alcaline et folate-C6-NHS-phosphatase alcaline

0,5 mL d'une solution de phosphatase alcaline (PAL) recombinante à 20 mg/mL (Roche, Ref. 03-535-452), sont dialysés en boyau Spectra/Por® (seuil de coupure 6000-8000 Da, Spectrum Laboratories, USA) contre 500 mL de tampon carbonate 100 mM pH 8,3, sous agitation magnétique, pendant une nuit, à +2/8°C. En sortie de dialyse, la concentration de la protéine est déterminée par lecture de la densité optique à 280 nm et cette concentration est ajustée à 4 mg/mL.

Les esters activés folate-C4-NHS et folate-C6-NHS obtenus dans l'exemple 2 sont repris en DMSO à des concentrations de 0,39 mg/mL et de 0,5 mg/mL respectivement, en tenant compte de la pureté.

Pour un couplage de type (1-5) (1 mole de phosphatase alcaline-5 moles d'ester de folate), 1,125 mL de la solution de PAL sont mélangés avec 256 µL de la solution d'ester folate-C4-NHS d'une part et 255 µL de la solution d'ester folate-C6-NHS d'autre part. Le pourcentage de DMSO dans le milieu réactionnel est de 18,5%. Les mélanges sont incubés pendant une nuit à +2/8°C, en agitation sur une roue, dans des flacons bruns.

Ensuite, la réaction est bloquée par addition de lysine 10 mM diluée dans l'eau. La quantité de lysine ajoutée est équimolaire à la quantité d'ester utilisée pour le couplage. On ajoute donc 20,5 µL de la solution de lysine pour chacun des couplages. Les mélanges sont incubés pendant 20 minutes sur une roue, à +18/25°C.

Après arrêt de la réaction, 1 mL de chacun des conjugués sont dialysés en boyau Spectra/Por® (seuil de coupure environ 7000 Da) pendant 3h à +18/25°C contre 500 mL de tampon Tris 50 mM pH 7,4, NaCl 9 g/L, azide 0,9 g/L, sous agitation magnétique. Au bout de 3 heures, les boyaux sont transférés dans de nouveaux bains contenant toujours 500 mL du même tampon. La dialyse est poursuivie sur la nuit à +2/8°C, sous agitation magnétique.

En sortie de dialyse, du tampon de conservation 10x (Tris 500 mM pH 7,4, NaCl 90 g/L, MgCl₂ 50 mM, ZnCl₂ 1 mM, SDS 0,01%, azide 9 g/L) est ajouté aux volumes récupérés des boyaux. Le volume obtenu est d'environ 1 mL par conjugué. A la suite de la dialyse, les conjugués ne sont que semi-purifiés : la dialyse permet d'éliminer le folate libre, n'ayant pas réagi, mais pas la phosphatase alcaline libre. Les conjugués peuvent être utilisés dans un immunodosage à ce stade et c'est ce qui a été fait dans l'exemple 6 infra.

Pour éliminer la PAL libre et ainsi obtenir des conjugués avec une pureté améliorée, la chromatographie d'interactions hydrophobes a été réalisée en utilisant une colonne RESOURCE Phenyl (Cat No. 17-1186-01, GE Healthcare Lifesciences) montée sur une chaîne de chromatographie ÄKTA. Le débit de la pompe est réglé à 2 mL/min. Le tampon TA est du Tris 50 mM pH 7,4, NACl 9 g/L, MgCl₂ 5 mM, ZnCl₂ 0,1 mM, azide 0,9 g/L, (NH₄)₂SO₄ 1,6 M. Le tampon TB est Tris 50 mM pH 7,4, NaCl 9 g/L, MgCl₂ 5 mM, ZnCl₂ 0,1 mM, azide 0,9 g/L. La colonne RESOURCE Phenyl est équilibrée en tampon TA. Le conjugué à purifier est mélangé volume pour volume avec le tampon TB. Puis, on ajoute 2 volumes du tampon Tris 50 mM pH 7,4, NaCl 9 g/L, MgCl₂ 5 mM, ZnCl₂ 0,1 mM, azide 0,9 g/L, (NH₄)₂SO₄ 3,2 M. Cette étape permet d'avoir le conjugué dans le tampon TA. L'injection du conjugué (628 µL pour folate-C4-NHS-PAL et 560 µL pour folate-C6-NHS-PAL, sur une boucle de 5 mL) est suivie d'un lavage de 20 mL en tampon TA. Ensuite un gradient de 0 à 57% de TB est appliqué pendant 30 mL, puis un lavage en 57% de tampon TB pendant 20 mL. Cette étape est suivie par un deuxième gradient de 57 à 100% de TB appliqué pendant 30 mL, puis par un lavage en tampon TB pendant 20 mL. La dernière étape consiste en un gradient de 0 à 100% en eau pendant 20 mL, et puis un lavage en eau pendant 20 mL. Le déroulement de la chromatographie est suivi par mesure de la densité optique à 280 nm. Les fractions à partir de 74 mL d'élution jusqu'à 104 mL (soit au total 30 mL) sont récupérées, rassemblées puis concentrées par diafiltration en utilisant une cellule Amicon (Amicon stirred cells, Millipore), une membrane Amicon PM avec un seuil de coupure de 10 000 Da et le tampon TB. Lors de cette étape, le volume de la solution de conjugué est réduit à environ 0,5 mL. Les conjugués sont conservés à +2/8°C jusqu'à leur utilisation dans un immunodosage.

### Exemple 6: Dosage de la vitamine B9 en utilisant les conjugués folate-C4-NHS-phosphatase alcaline et folate-C6-NHS-phosphatase alcaline et comparaison avec le conjugué du dosage Axsym (Abbott Laboratories)

Les dosages immunologiques ont été réalisés en utilisant l'automate d'immunoanalyse VIDAS® (bioMérieux). Le cône à usage unique sert à la fois de phase solide pour la réaction et de système de pipetage. La cartouche est composée de 10 puits recouverts d'une feuille d'aluminium scellée et étiquetée. Le premier puits comporte une partie prédécoupée pour faciliter l'introduction de l'échantillon. Le dernier puits est une cuvette optique dans laquelle la fluorescence du substrat est mesurée. Les différents réactifs nécessaires à l'analyse sont contenus dans les puits intermédiaires.

### a) Sensibilisation et passivation des cônes

Les cônes ont été sensibilisés avec 300 µL d'un anticorps monoclonal de souris « anti-folate binding protein » (clone P8C5E4, bioMérieux) dilué à 5 µg/mL dans un tampon tris 0,2 M, pH 6,2. Après 6 heures d'incubation à +18/25°C, un lavage est effectué avec une solution de NaCl 1 M. Ensuite, 300 µL d'une solution de « folate binding protein » (FBP, Cat. No. F0524, Scripps Laboratories) diluée à 6 ng/mL dans un tampon phosphate 100 mM, pH 7,4 NaCl 0,15 M contenant de l'albumine humaine et un sucre sont ajoutés. La sensibilisation / passivation se poursuit à +18/25°C sur la nuit. Les cônes sont vidés, séchés puis conservés à +4°C jusqu'à utilisation.

### b) Préparation d'une gamme à partir d'échantillons biologiques

Des échantillons de sérum humain contenant différentes concentrations de folate ont été obtenus auprès du laboratoire Biomnis (Lyon, France). Les échantillons ayant les mêmes concentrations ont été mélangés afin d'augmenter le volume disponible par point de gamme. Les mélanges ont ensuite été aliquotés en 120 µL et congelés à -20°C jusqu'à utilisation. Les concentrations nominales de chacun des points sont : 1,3 ng/mL - 4,4 ng/mL - 10 ng/mL - 20 ng/mL. Le point de gamme 0 ng/mL a été préparé en dissolvant 10% d'albumine sérique humaine en tampon phosphate borate 10 mM pH 8,5, 0,15 M NaCl.

### c) Extraction des échantillons

L'objectif de l'étape d'extraction est de dissocier le folate sérique de ses partenaires de liaison et de le rendre accessible pour le dosage. A 250 µL d'échantillon sont ajoutés 50 µL d'une solution de TCEP à 62,5 mg/mL (tris(2-carboxyethyl)phosphine) et 250 µL d'une solution de NaOH 0,8N+KCN 0,005%. Le mélange est incubé à +18/25°C pendant 15 minutes, à l'abri de la lumière. Après cette étage, 1 mL de tampon glycine 1M pH4 est ajouté.

### d) Mode opératoire de la réaction d'immunodosage

L'échantillon à doser (200 µL), extrait selon le protocole décrit en c), est introduit dans le premier puits de la cartouche. Ensuite toutes les étapes de la réaction de dosage sont réalisées automatiquement par le VIDAS®. Les cônes préparés selon le protocole décrit en a) sont mouillés par un tampon glycine 1 M pH 10, NaCl 0,1 M et saccharose 2%. L'échantillon à doser est mélangé avec 200 µL d'une dilution de conjugué qui est un dérivé de folate marqué à la phosphatase alcaline. Le mélange échantillon/conjugué est incubé dans le cône pendant environ 20 minutes, durant lesquelles il s'effectue une compétition entre les folates présents dans l'échantillon et le dérivé de folate du conjugué vis-à-vis des sites de la protéine FBP présentée sur le cône. Ensuite, 3 lavages successifs avec un tampon Tris 100 mM pH 7,4, NaCl 0,15 M, Tween® 20 0,1% sont effectués afin d'éliminer les composés non fixés. Lors de l'étape finale de révélation, le substrat 4-méthylombelliferyl phosphate est aspiré puis refoulé dans le cône ; l'enzyme du conjugué catalyse la réaction d'hydrolyse de ce substrat en 4-méthylombelliferone dont la fluorescence émise est mesurée à 450 nm. La valeur du signal de fluorescence est inversement proportionnelle à la concentration de folate présent dans l'échantillon.

Dans l'expérience présentée dans le Tableau 1, trois conjugués ont été comparés. Il s'agit des deux conjugués obtenus dans l'exemple 3 et, en tant que référence, le conjugué utilisé dans le kit Abbott Axsym Folate (Cat. No. B7K460, Abbott Laboratories).
(i) les conjugués folate-C4-PAL et folate-C6-PAL ont été dilués à une concentration entre 0,50 - 0,75 ng/mL dans le tampon diluant conjugué qui contient 100 mM de Tris pH 8,5, 0,15 M NaCl, 20 mg/L d'IgG de souris, des agents de stabilisation, des agents de préservation et d'autres additifs.
(ii) le conjugué du kit Axsym folate est un conjugué d'acide ptéroïque (analogue de folate) et de phosphatase alcaline. Ce conjugué a été dilué au 1/80 le tampon diluant conjugué avant utilisation dans le VIDAS®.

Les points de gamme préparés en b) ont été mesurés avec chaque format de dosage. Le Tableau 1 ci-dessous résume les résultats obtenus en signal RFV (=relative fluorescence value) et ratio B/B0%. Le ratio B/B0% est le signal obtenu pour le point de gamme testé divisé par le signal obtenu pour le point de gamme 0 ng/mL de folate, multiplié par 100.

**Tableau 1**

| | REF = conjugué Axsym | | Folate-C4-PAL | | Folate-C6-PAL | |
|---|---|---|---|---|---|---|
| [c] folate (ng/mL) | Signal (RFV) | B/B0% | Signal (RFV) | B/B0% | Signal (RFV) | B/B0% |
| 0 | 3399 | 100 | 3544 | 100 | 4118 | 100 |
| 1,3 | 3329 | 98 | 3095 | 87 | 3563 | 87 |
| 4,4 | 2442 | 72 | 2016 | 57 | 2619 | 64 |
| 10 | 925 | 27 | 995 | 28 | 1131 | 27 |
| 20 | 39 | 1 | 24 | 1 | 34 | 1 |

Une diminution du signal de 87% est observée dès 1,3 ng/mL de folate avec les conjugués folate-C4-PAL et folate-C6-PAL de l'invention, alors qu'avec le conjugué de référence la diminution du signal commence à peine.

Par conséquent, les dosages utilisant les conjugués folate-C4-PAL et folate-C6-PAL sont plus sensibles que le dosage utilisant le conjugué de référence, et permettent une meilleure détection et quantification des concentrations inférieures à 4,4 ng/mL, et même inférieures à 1,3 ng/mL.

Concernant les dérivés et conjugués de de folate selon l'invention, en particulier ceux répondant aux formules générales (I), (I'), (III), (III'), (III"), (VI), (VI'), (VIII) et (IX) susvisées, il convient de noter que, même si la partie ptérine de ceux-ci est représentée schématiquement sous sa forme cétone, la présente invention couvre bien évidemment toutes les - et chacune des - formes tautomères desdits dérivés et conjugués de folate susceptibles d'être obtenues au niveau de ladite partie ptérine, et en particulier la forme 2-amino-4-hydroxy-6-méthylptéridine.

### Références Bibliographiques

1. Antony AC, The biological chemistry of folate receptors, Department of Medicine, Indiana University School of Medicine, Indianapolis, Blood. 1992 Jun 1;79(11):2807-20
2. Reif VD, Reamer JT, Grady LT., Chromatic assays for folie acid, J Pharm Sci. 1977 Aug:66(8):1112-6 PMID:894496
3. Dueker SR, Lin Y, Jones AD, Mercer R, Fabbro E, Miller JW, Green R, Clifford AJ., Détermination of blood folate using acid extraction and internally standardized gas chromatography-mass spectrometry detection, Anal Biochem. 2000 Aug 1;283(2):266-75 PMID:10906248
4. Pfeiffer CM, Fazili Z, McCoy L, Zhang M, Gunter EW, Détermination of folate vitamers in human serum by stable-isotope-dilution tandem mass spectrometry and comparison with radioassay and microbiologie assay, Clin Chem. 2004 Feb;50(2):423-32. Epub 2003 Dec 11 PMID:14670827
5. Waxman S. et Schreiber C., Détermination of folate by use of radioactive folate and binding proteins, Methods Enzymol. 1980; 66:468-83. No abstract available. PMID:7374487
6. Hansen, S. I. et Holm, J., A compétitive enzyme-linked ligand sorbent assay (ELLSA) for quantitation of folates, Anal Biochem. 1988 Jul;172(1):160-4. PMID:3189760
7. Owen, W.E. et Roberts, W. L., Comparison of five automated serum and whole blood folate assays, Am J Clin Pathol. 2003 Jul;120(1):121-6. PMID:12866382
8. Arcot J. et Shrestha A., Folate : methods of analysis, Food Science and Technology, School of Chemical Engineering and Industrial Chemistry, The University of New South Wales, Sydney, Australia, 2005

## Revendications

1. Utilisation d'un dérivé de folate pour doser le/les folate(s) *in vitro* dans un échantillon, tel qu'un échantillon biologique, ledit dosage étant un immunodosage par compétition non radio-isotopique, et ledit dérivé de folate répondant à la formule générale (I) : 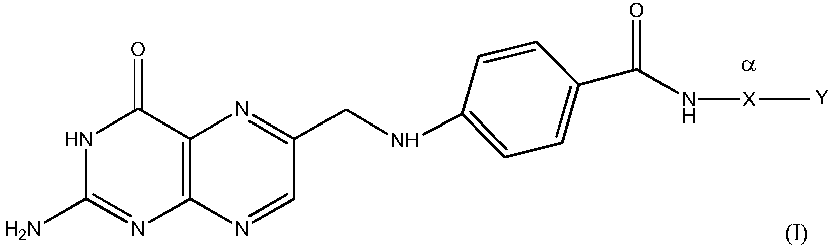 dans laquelle :
- X est une chaîne hydrocarbonée aliphatique contenant de 1 à 10 atomes de carbone, ladite chaîne hydrocarbonée aliphatique ne contenant que de l'hydrogène et du carbone ; et
- Y représente un groupement fonctionnel adapté pour permettre le couplage avec une molécule distincte M, telle qu'une protéine, ledit couplage comprenant la formation d'au moins une liaison covalente entre Y et un groupement fonctionnel porté par ladite molécule distincte M, Y étant un groupement de type centre électrophile ou un groupement de type centre nucléophile, de préférence un groupement de type centre électrophile, adapté pour permettre la formation d'une liaison amide, ester, ou thioester, de préférence amide ou ester, avantageusement amide, entre Y et le groupement fonctionnel porté par ladite molécule distincte M.

2. Utilisation selon la revendication 1, dans laquelle X est une chaîne hydrocarbonée contenant de 2 à 7 atomes de carbone, de préférence de 3 à 5 atomes de carbone, avantageusement X est une chaîne hydrocarbonée de 3 atomes de carbone ou de 5 atomes de carbone.

3. Utilisation selon la revendication 1 ou 2, dans laquelle X est une chaîne hydrocarbonée saturée.

4. Utilisation selon l'une des revendications 1 à 3, dans laquelle X est une chaîne hydrocarbonée linéaire.

5. Utilisation selon l'une des revendications 1 à 4, dans laquelle Y est un groupement de type centre électrophile, répondant à la formule générale (II) suivante : 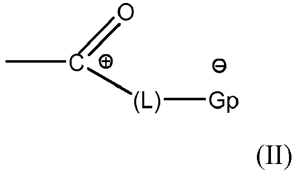 dans laquelle Gp est un groupement partant, éventuellement relié à la fonction carbonyle par un bras L, Gp étant adapté pour être dissocié du groupement de type centre électrophile lors d'une réaction avec un groupement nucléophile porté par ladite molécule distincte M, tel qu'une amine primaire.

6. Utilisation selon la revendication 5, dans laquelle le groupement (L)-Gp est choisi parmi les groupements adaptés pour permettre la formation d'une liaison amide, ester, ou thioester, de préférence amide ou ester, avantageusement amide, entre ledit groupement de type centre électrophile et un groupement fonctionnel porté par ladite molécule distincte M.

7. Utilisation selon la revendication 5 ou 6, dans laquelle le groupement (L)-Gp est choisi parmi : -OH, -NH-(CH₂)ₘ-COOH, -N₃, 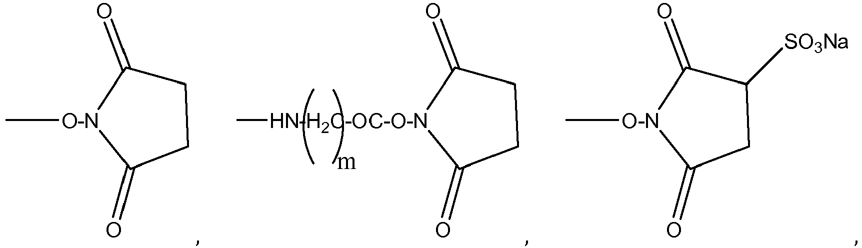 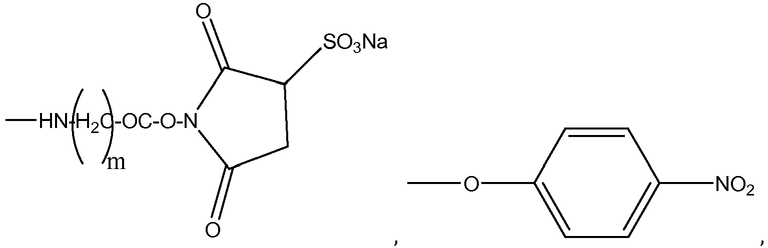 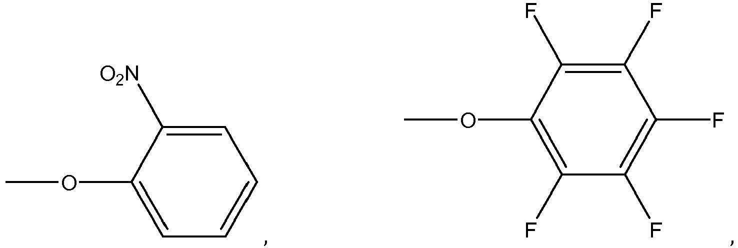 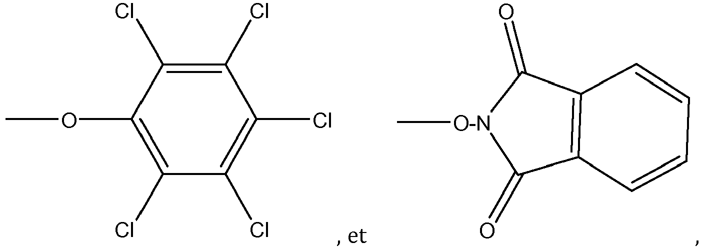 m étant un nombre entier compris entre 1 et 10.

8. Utilisation selon la revendication 7, dans laquelle m est compris entre 1 et 5, avantageusement entre 1 et 3.

9. Utilisation selon l'une des revendications 5 à 8, dans laquelle le groupement (L)-Gp est choisi parmi :
-OH et -NH-(CH₂)ₘ-COOH et 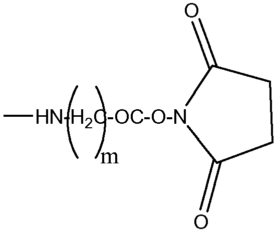
, m étant compris entre 1 et 10 et de préférence m étant égal à 1.

10. Utilisation selon l'une des revendications 1 à 9, dans laquelle le dérivé de formule générale (I) est utilisé sous forme de conjugué avec ladite molécule M, et ledit conjugué étant représenté par la formule générale (III) suivante : 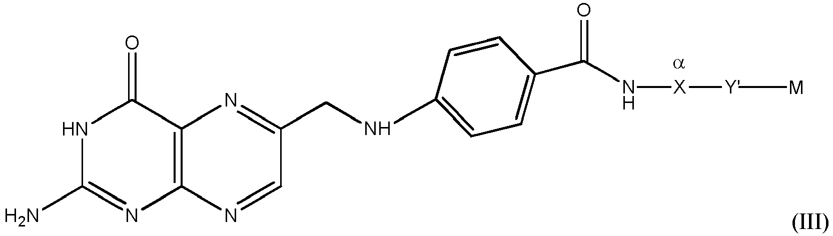
dans laquelle X et M sont tels que définis dans l'une des revendications 1 à 9, et dans laquelle Y' est un dérivé du groupement fonctionnel Y après couplage du dérivé de formule générale (I) avec ladite molécule M,
ladite molécule M étant de préférence une molécule marqueur Mm.

11. Utilisation selon la revendication 10, dans laquelle Y' est représenté par la formule générale (IV) suivante : 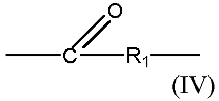 ou par la formule générale (V) suivante : 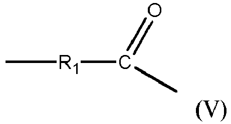
dans lesquelles R1 est -NH-, -O-, ou -S-, de préférence -NH- ou -O-, avantageusement -NH- ;
de préférence Y' étant représenté par la formule générale (IV).

12. Procédé de dosage *in vitro* du/des folate(s) dans un échantillon, tel qu'un échantillon biologique, ledit dosage étant un immunodosage par compétition non radio-isotopique, ledit procédé comprenant les étapes suivantes :
a) mettre en présence, au sein dudit échantillon biologique, (i) au moins un partenaire de liaison du/des folate(s), tel qu'un anticorps adapté pour se lier au(x) folate(s) ou tel que le récepteur des folates, avec (ii) au moins un composé choisi parmi un dérivé de folate tel que défini dans l'une des revendications 1 à 9 et un conjugué tel que défini dans la revendication 10 ou 11, au moins l'un desdits composés (i) et (ii) étant adapté pour émettre un signal,
b) éventuellement laisser un laps de temps suffisant pour permettre la réaction de compétition,
c) mesurer l'intensité du signal et en déduire la concentration en folate(s) présente dans ledit échantillon biologique par référence à une courbe de calibration établissant une relation entre intensité du signal mesurée et concentration en folate(s).

13. Procédé selon la revendication 12, **caractérisé en ce que** le composé (ii) est un conjugué tel que défini dans la revendication 10 ou 11.

14. Méthode de diagnostic *in vitro* visant à déterminer si un patient humain ou animal, de préférence humain, présente une carence en folate(s), ladite méthode comprenant les étapes suivantes :
a) au sein d'un échantillon biologique, de préférence de sang total, de plasma ou de sérum, prélevé à partir dudit patient, doser le/les folate(s) en mettant en oeuvre le procédé de dosage *in vitro* selon la revendication 12 ou 13, afin d'en déduire la concentration en folate(s) dans l'échantillon biologique
b) comparer ladite concentration avec une valeur seuil, correspondant à une concentration prédéterminée en folate(s) au-dessous de laquelle un patient est considéré comme carencé en folate(s), et
c) si la concentration dosée est inférieure à ladite valeur seuil, en déduire que le patient présente une carence en folate(s).

15. Dérivé de folate de formule générale (I), adapté pour être utilisé dans un immunodosage par compétition non radio-isotopique : 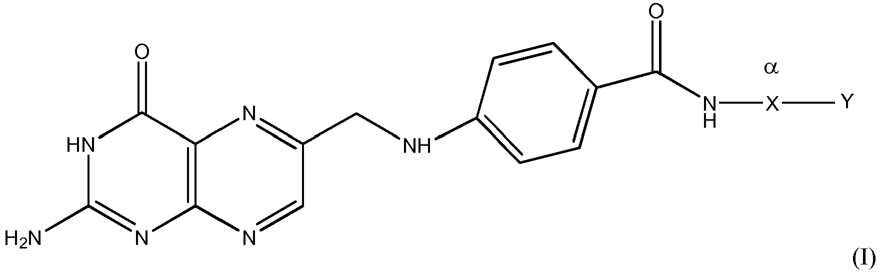 dans laquelle :
- X est telle que définie dans l'une des revendications 1 à 9;
- Y est un groupement de type centre électrophile ou de type centre nucléophile, de préférence un groupement de type centre électrophile, adapté pour permettre la formation d'une liaison amide, ester, ou thioester, de préférence amide ou ester, avantageusement amide, entre Y et un groupement fonctionnel porté par une molécule distincte M, ; et dans laquelle :
- lorsque X est une chaîne hydrocarbonée linéaire et saturée contenant un nombre d'atomes de carbone égal à 2, Y n'est pas un groupement -(O-CH₂-CH₂)₂-NH₂ ;
- lorsque Y est un groupement de type centre nucléophile consistant en une amine primaire, X comprend un nombre d'atomes de carbone différent de 6,
- lorsque Y est un groupement de type centre électrophile, ce dernier répond à la formule générale (II) suivante : 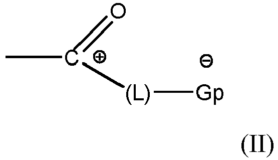
dans laquelle Gp est un groupement partant, éventuellement relié à la fonction carbonyle par un bras L, Gp étant adapté pour être dissocié du groupement de type centre électrophile lors d'une réaction avec un groupement nucléophile porté par ladite molécule distincte M, tel qu'une amine primaire, et dans lequel, lorsque L est absent et Gp est -OH, X comprend un nombre d'atomes de carbone supérieur à 4.

16. Dérivé de folate selon la revendication 15, dans lequel Y est un groupement de type centre nucléophile consistant en un groupement fonctionnel différent d'une amine primaire.

17. Dérivé de folate selon la revendication 15, dans laquelle Y est groupement de type centre électrophile répondant à la formule générale (II) et dans laquelle (L)-Gp est un groupement partant choisi parmi : -OH, -NH-(CH₂)ₘ-COOH, -N₃, 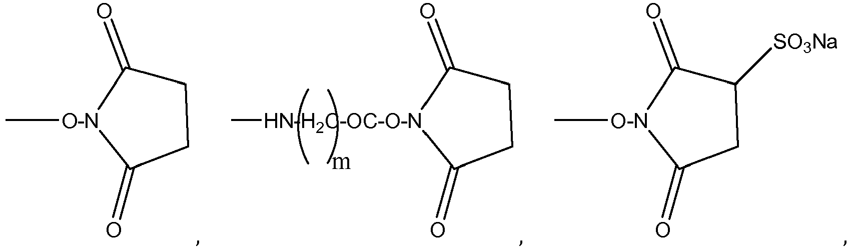 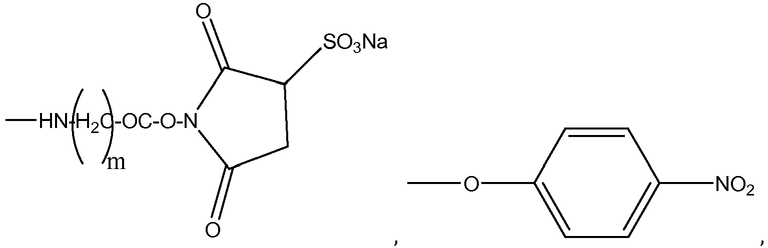 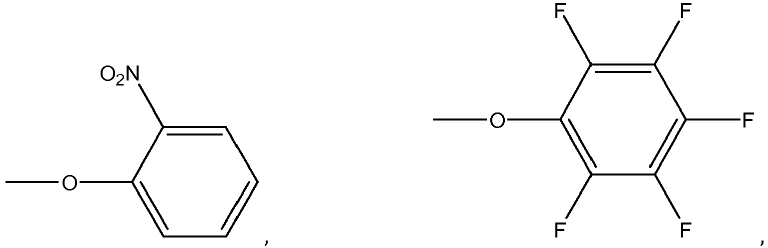 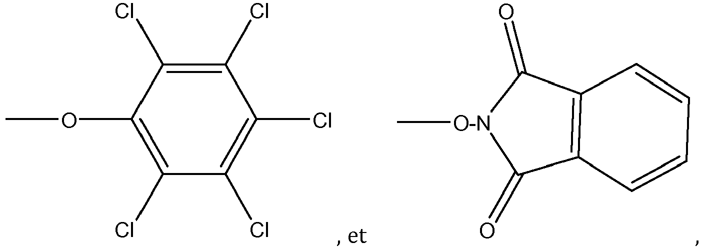 m étant un nombre entier compris entre 1 et 10, de préférence compris entre 1 et 5, avantageusement compris entre 1 et 3, dans lequel lorsque L est absent et Gp est -OH, X comprend un nombre d'atomes de carbone supérieur à 4.

18. Dérivé de folate selon la revendication 17, dans lequel (L)-Gp est choisi parmi :
-OH et -NH-(CH₂)ₘ-COOH et 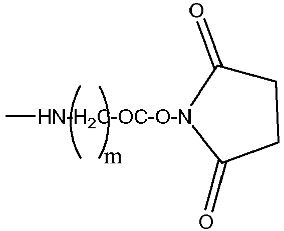
, m étant compris entre 1 et 10 et de préférence m étant égal à 1,
dans lequel lorsque L est absent et Gp est -OH, X comprend un nombre d'atomes de carbone supérieur à 4.

19. Dérivé de folate selon l'une des revendications 15, 17 ou 18, dans lequel Gp est un groupement différent d'un groupement -OH.

20. Conjugué comprenant un dérivé de folate selon l'une des revendications 15 à 19 et une molécule distincte M, ledit dérivé et ladite molécule M étant liés par au moins une liaison amide, ester, ou thioester, de préférence une liaison amide ou ester, avantageusement par une liaison amide, entre Y et un groupement fonctionnel porté par ladite molécule M.

21. Conjugué selon la revendication 20, dans lequel ladite molécule M est une molécule marqueur Mₘ, permettant le marquage direct ou indirect dudit dérivé de folate.

22. Kit permettant la mise en oeuvre du procédé selon la revendication 12 ou 13, ledit kit comprenant :
- (i) au moins un partenaire de liaison du ou des folate(s) tel qu'un anticorps adapté pour se lier au(x) folate(s) ou tel que le récepteur des folates,
- (ii) au moins un composé choisi parmi un dérivé de folate tel que défini dans l'une des revendications 1 à 9 et un conjugué tel que défini dans la revendication 10 ou 11,
au moins l'un desdits composés (i) et (ii) étant adapté pour émettre un signal, et
- au moins un moyen de calibration.

## Patentansprüche

1. Eine Verwendung eines Folatderivats zum Testen des Folats/der Folate in vitro in einer Probe, wie etwa einer biologischen Probe, wobei es sich bei dem Test um einen nicht radioisotopischen kompetitiven Immunoassay handelt und das Folatderivat der folgenden allgemeinen Formel (I) entspricht: 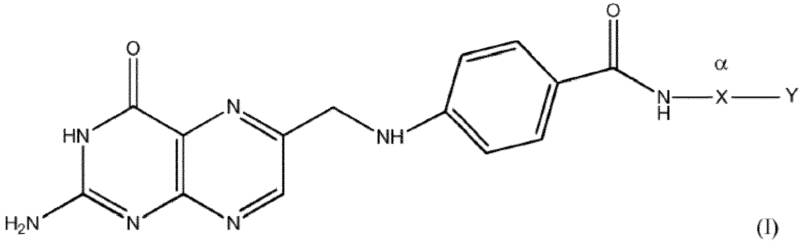 wobei:
- X eine aliphatische Kohlenwasserstoffkette, enthaltend 1 bis 10 Kohlenstoffatome, ist, wobei die aliphatische Kohlenwasserstoffkette nur Wasserstoff und Kohlenstoff enthält; und
- Y eine funktionelle Gruppe darstellt, die geeignet ist, um das Koppeln an ein separates Molekül M, wie etwa ein Protein, zu ermöglichen, wobei das Koppeln die Bildung von mindestens einer kovalenten Bindung zwischen Y und einer funktionellen Gruppe beinhaltet, die von dem separaten Molekül M getragen wird, wobei Y eine Gruppe vom Typ elektrophiles Zentrum oder eine Gruppe vom Typ nucleophiles Zentrum, vorzugsweise eine Gruppe vom Typ elektrophiles Zentrum, ist, die geeignet ist, um die Bildung einer Amid-, Ester- oder Thioesterbindung, vorzugsweise einer Amid- oder Esterbindung, vorteilhafterweise einer Amidbindung, zwischen Y und der funktionellen Gruppe, die von dem separaten Molekül M getragen wird, zu ermöglichen.

2. Verwendung gemäß Anspruch 1, wobei X eine Kohlenwasserstoffkette, enthaltend 2 bis 7 Kohlenstoffatome, vorzugsweise 3 bis 5 Kohlenstoffatome, ist, wobei X vorteilhafterweise eine Kohlenwasserstoffkette mit 3 Kohlenstoffatomen oder 5 Kohlenstoffatomen ist.

3. Verwendung gemäß Anspruch 1 oder 2, wobei X eine gesättigte Kohlenwasserstoffkette ist.

4. Verwendung gemäß einem der Ansprüche 1 bis 3, wobei X eine lineare Kohlenwasserstoffkette ist.

5. Verwendung gemäß einem der Ansprüche 1 bis 4, wobei Y eine Gruppe vom Typ elektrophiles Zentrum ist, die der folgenden allgemeinen Formel (II) entspricht: 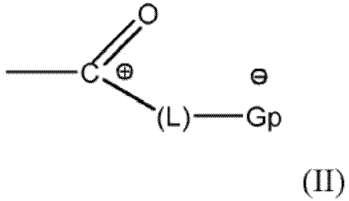 wobei Gp eine Abgangsgruppe ist, die optional durch einen Arm L an die Carbonylfunktion gebunden ist, wobei Gp geeignet ist, während einer Reaktion mit einer von dem separaten Molekül M getragenen nucleophilen Gruppe, wie etwa einem primären Amin, von der Gruppe vom Typ elektrophiles Zentrum getrennt zu werden.

6. Verwendung gemäß Anspruch 5, wobei die Gruppe (L)-Gp aus den Gruppen ausgewählt ist, die geeignet sind, die Bildung einer Amid-, Ester- oder Thioesterbindung, vorzugsweise einer Amid- oder Esterbindung, vorteilhafterweise einer Amidbindung, zwischen der Gruppe vom Typ elektrophiles Zentrum und einer funktionellen Gruppe, die von dem separaten Molekül M getragen wird, zu ermöglichen.

7. Verwendung gemäß Anspruch 5 oder 6, wobei die Gruppe (L)-Gp aus Folgendem ausgewählt ist: -OH, -NH-(CH₂)ₘ-COOH, -N₃, 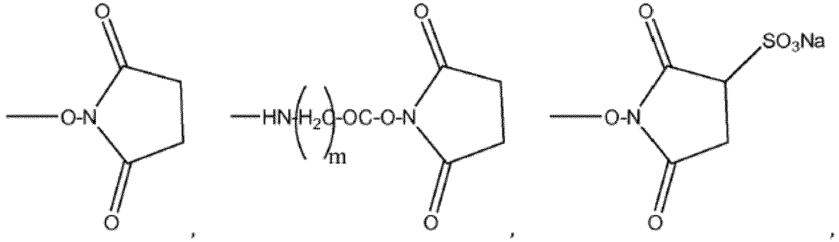 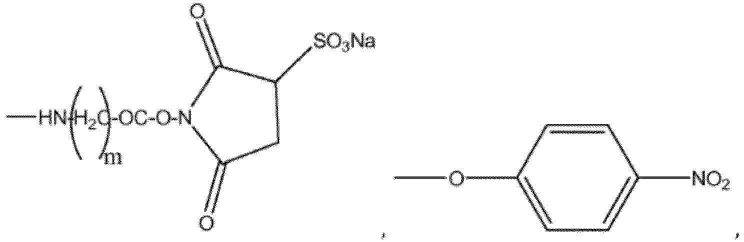 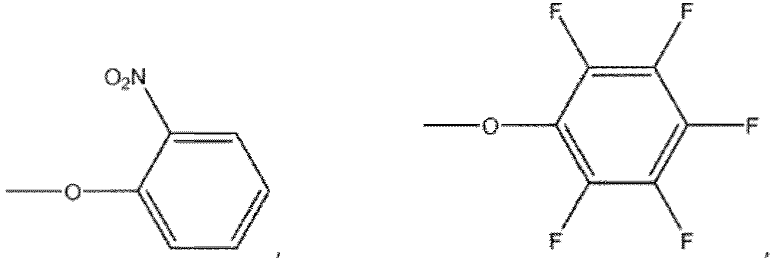 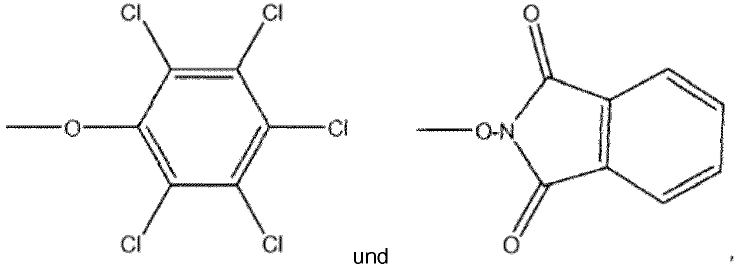 wobei m eine ganze Zahl von 1 bis 10 ist.

8. Verwendung gemäß Anspruch 7, wobei m von 1 bis 5, vorteilhafterweise von 1 bis 3 beträgt.

9. Verwendung gemäß einem der Ansprüche 5 bis 8, wobei die Gruppe (L)-Gp aus Folgendem ausgewählt ist:
-OH und -NH-(CH₂)ₘ-COOH und 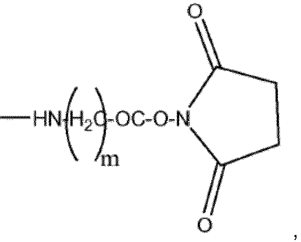
wobei m von 1 bis 10 beträgt und m vorzugsweise gleich 1 ist.

10. Verwendung gemäß einem der Ansprüche 1 bis 9, wobei das Derivat der allgemeinen Formel (I) in Form eines Konjugats mit dem Molekül M verwendet wird und das Konjugat durch die folgende allgemeine Formel (III) dargestellt ist: 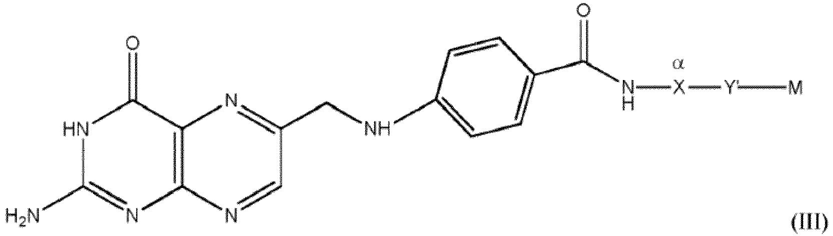
wobei X und M wie in einem der Ansprüche 1 bis 9 definiert sind und wobei Y' ein Derivat der funktionellen Gruppe Y nach Kopplung des Derivats der allgemeinen Formel (I) an das Molekül M ist,
wobei das Molekül M vorzugsweise ein Markermolekül Mm ist.

11. Verwendung gemäß Anspruch 10, wobei Y' durch die folgende allgemeine Formel (IV) dargestellt ist: 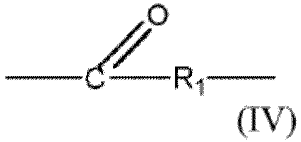 oder durch die folgende allgemeine Formel (V): 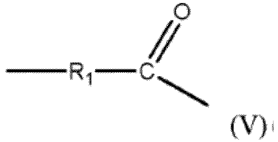
wobei R1 -NH-, -O- oder-S-, vorzugsweise -NH- oder-O-, vorteilhafterweise -NH-, ist;
wobei Y' vorzugsweise durch die allgemeine Formel (IV) dargestellt ist.

12. Ein Verfahren zum In-vitro-Testen von Folat(en) in einer Probe, wie etwa einer biologischen Probe, wobei es sich bei dem Test um einen nicht radioisotopischen kompetitiven Immunoassay handelt, wobei das Verfahren die folgenden Schritte beinhaltet:
a) In-Kontakt-Bringen, in der biologischen Probe, von (i) mindestens einem Bindungspartner des Folats/der Folate, wie etwa einem Antikörper, der geeignet ist, um an das Folat/die Folate zu binden, oder wie etwa dem Folatrezeptor, mit (ii) mindestens einer Verbindung, die ausgewählt ist aus einem Folatderivat, wie in einem der Ansprüche 1 bis 9 definiert, und einem Konjugat, wie in Anspruch 10 oder 11 definiert, wobei mindestens eine der Verbindungen (i) und (ii) zum Aussenden eines Signals geeignet ist,
b) optional genügend Zeit lassen, um die kompetitive Reaktion zu ermöglichen,
c) Messen der Intensität des Signals und Ableiten der Konzentration an Folat(en), die in der biologischen Probe vorhanden ist, unter Bezugnahme auf eine Kalibrierungskurve, die eine Beziehung zwischen der gemessenen Signalintensität und der Konzentration an Folat(en) herstellt.

13. Verfahren gemäß Anspruch 12, **dadurch gekennzeichnet, dass** die Verbindung (ii) ein Konjugat wie in Anspruch 10 oder 11 definiert ist.

14. Eine In-vitro-Diagnosemethode, die darauf abzielt zu bestimmen, ob ein menschlicher oder tierischer Patient, vorzugsweise ein menschlicher Patient, einen Mangel an Folat(en) aufweist, wobei die Methode die folgenden Schritte beinhaltet:
a) Testen, in einer biologischen Probe, vorzugsweise Vollblut, Plasma oder Serum, die von dem Patienten genommen wurde, des Folats/der Folate durch Einsetzen des In-vitro-Testverfahrens gemäß Anspruch 12 oder 13, um die Konzentration an Folat(en) in der biologischen Probe abzuleiten,
b) Vergleichen der Konzentration mit einem Schwellenwert, der einer vorher festgelegten Konzentration an Folat(en) entspricht, unter der ein Patient als an einem Mangel an Folat(en) leidend gilt, und
c) Ableiten, wenn die gemessene Konzentration niedriger als der Schwellenwert ist, dass der Patient einen Mangel an Folat(en) aufweist.

15. Ein Folatderivat der allgemeinen Formel (I), das zur Verwendung in einem nicht radioisotopischen kompetitiven Immunoassay geeignet ist: 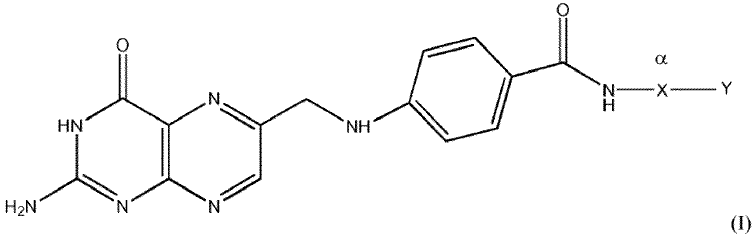 wobei:
- X wie in einem der Ansprüche 1 bis 9 definiert ist;
- Y eine Gruppe vom Typ elektrophiles Zentrum oder eine Gruppe vom Typ nucleophiles Zentrum, vorzugsweise eine Gruppe vom Typ elektrophiles Zentrum, ist, die geeignet ist, um die Bildung einer Amid-, Ester- oder Thioesterbindung, vorzugsweise einer Amid- oder Esterbindung, vorteilhafterweise einer Amidbindung, zwischen Y und einer funktionellen Gruppe, die von einem separaten Molekül M getragen wird, zu ermöglichen; und wobei:
- wenn X eine lineare und gesättigte Kohlenwasserstoffkette ist, die eine Anzahl von Kohlenstoffatomen von gleich 2 enthält, Y keine -(O-CH₂-CH₂)₂-NH₂-Gruppe ist;
- wenn Y eine Gruppe vom Typ eines nucleophilen Zentrums ist, die aus einem primären Amin besteht, X eine Anzahl von Kohlenstoffatomen, die nicht 6 ist, beinhaltet,
- wenn Y eine Gruppe vom Typ elektrophiles Zentrum ist, letztere der folgenden allgemeinen Formel (II) entspricht: 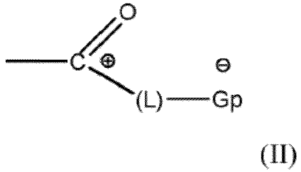
wobei Gp eine Abgangsgruppe ist, die optional durch einen Arm L an die Carbonylfunktion gebunden ist, wobei Gp geeignet ist, während einer Reaktion mit einer von dem separaten Molekül M getragenen nucleophilen Gruppe, wie etwa einem primären Amin, von der Gruppe vom Typ elektrophiles Zentrum getrennt zu werden, und wobei, wenn L abwesend ist und Gp -OH ist, X eine Anzahl von Kohlenstoffatomen von mehr als 4 beinhaltet.

16. Folatderivat gemäß Anspruch 15, wobei Y eine Gruppe vom Typ eines nucleophilen Zentrums ist, die aus einer anderen funktionellen Gruppe als einem primären Amin besteht.

17. Folatderivat gemäß Anspruch 15, wobei Y eine Gruppe vom Typ elektrophiles Zentrum ist, die der allgemeinen Formel (II) entspricht, und wobei (L)-Gp eine Abgangsgruppe ist, die ausgewählt ist aus: -OH, -NH-(CH₂)ₘ-COOH, -N₃, 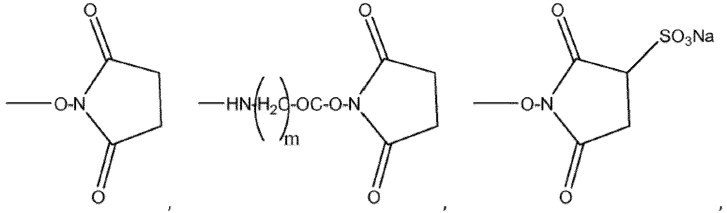 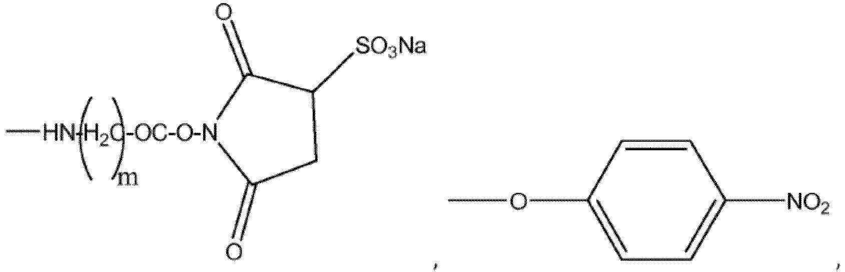 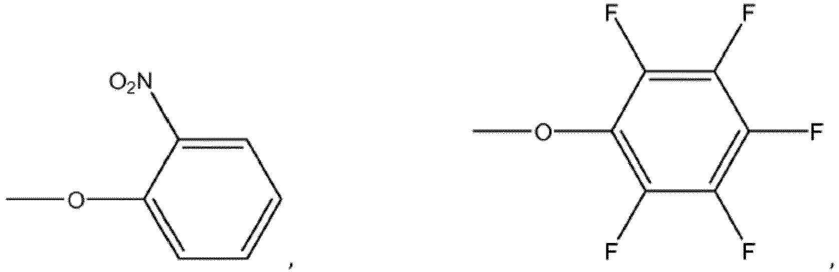 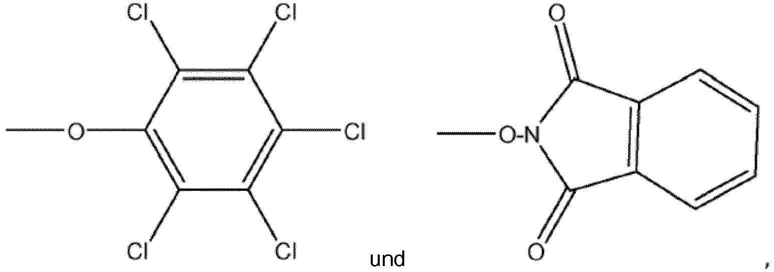 wobei m eine ganze Zahl von 1 bis 10, vorzugsweise von 1 bis 5, vorteilhafterweise von 1 bis 3, ist, wobei, wenn L abwesend ist und Gp -OH ist, X eine Anzahl von Kohlenstoffatomen von mehr als 4 beinhaltet.

18. Folatderivat gemäß Anspruch 17, wobei (L)-Gp aus Folgendem ausgewählt ist: -OH und -NH-(CH₂)ₘ-COOH und 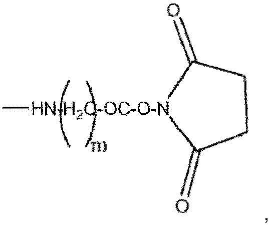
wobei m von 1 bis 10 beträgt und m vorzugsweise gleich 1 ist.
wobei, wenn L abwesend ist und Gp -OH ist, X eine Anzahl von Kohlenstoffatomen von mehr als 4 beinhaltet.

19. Folatderivat gemäß einem der Ansprüche 15, 17 oder 18, wobei Gp eine andere Gruppe als eine -OH-Gruppe ist.

20. Ein Konjugat, das ein Folatderivat gemäß einem der Ansprüche 15 bis 19 und ein separates Molekül M beinhaltet, wobei das Derivat und das Molekül M durch mindestens eine Amid-, Ester- oder Thioesterbindung, vorzugsweise eine Amid- oder Esterbindung, vorteilhafterweise durch eine Amidbindung, zwischen Y und einer von dem Molekül M getragenen funktionellen Gruppe gebunden sind.

21. Konjugat gemäß Anspruch 20, wobei das Molekül M ein Markermolekül Mₘ ist, das die direkte oder indirekte Markierung des Folatderivats ermöglicht.

22. Ein Kit, das den Einsatz des Verfahrens gemäß Anspruch 12 oder 13 ermöglicht, wobei das Kit Folgendes beinhaltet:
- (i) mindestens einen Bindungspartner des Folats/der Folate, wie etwa einen Antikörper, der zum Binden an das Folat/die Folate geeignet ist, oder wie etwa einen Folatrezeptor,
- (ii) mindestens eine Verbindung, die ausgewählt ist aus einem Folatderivat, wie in einem der Ansprüche 1 bis 9 definiert, und einem Konjugat, wie in Anspruch 10 oder 11 definiert,
wobei mindestens eine der Verbindungen (i) und (ii) geeignet ist, ein Signal auszusenden,
und
- mindestens ein Kalibrierungsmittel.

## Claims

1. Use of a folate derivative to assay *in vitro* the folate(s) in a sample, such as a biological sample, said assay being a non-radioisotopic competition immunoassay, and said folate derivative corresponding to general formula (I): 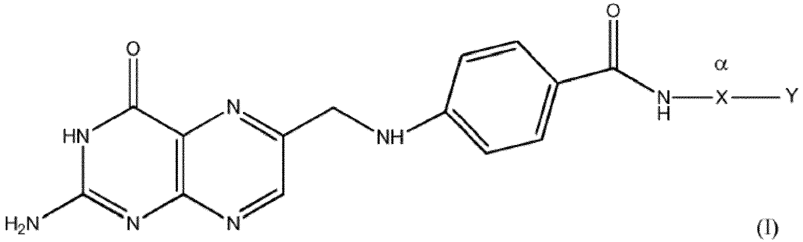 wherein:
- X is an aliphatic hydrocarbon chain containing from 1 to 10 carbon atoms, said aliphatic hydrocarbon chain containing only hydrogen and carbon; and
- Y represents a functional group suitable for enabling bonding to a separate molecule M, such as a protein, said bonding comprising the formation of at least one covalent bond between Y and a functional group carried by said separate molecule M, Y being an electrophilic centre type group or a nucleophilic centre type group, preferably an electrophilic centre type group, suitable for enabling the formation of an amide, ester, or thioester bond, preferably amide or ester, advantageously amide, between Y and the functional group carried by said separate molecule M.

2. Use according to claim 1, wherein X is a hydrocarbon chain containing from 2 to 7 carbon atoms, preferably from 3 to 5 carbon atoms, advantageously X is a hydrocarbon chain of 3 carbon atoms or 5 carbon atoms.

3. Use according to claim 1 or 2, wherein X is a saturated hydrocarbon chain.

4. Use according to one of claims 1 to 3, wherein X is a linear hydrocarbon chain.

5. Use according to one of claims 1 to 4, wherein Y is an electrophilic centre type group, corresponding to the following general formula (II): 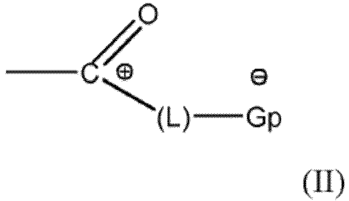 wherein Gp is a leaving group, possibly bound to the carbonyl function by an L arm, Gp being suitable for being dissociated from the electrophilic centre type group in a reaction with the nucleophilic group carried by said separate molecule M, such as a primary amine.

6. Use according to claim 5, wherein the (L)-Gp group is selected from the groups suitable for enabling the formation of an amide, ester, or thioester bond, preferably amide or ester, advantageously amide, between said electrophilic centre type group and a functional group carried by said separate molecule M.

7. Use according to claim 5 or 6, wherein the (L)-Gp group is selected from: -OH,
-NH-(CH₂)ₘ-COOH, -N₃, 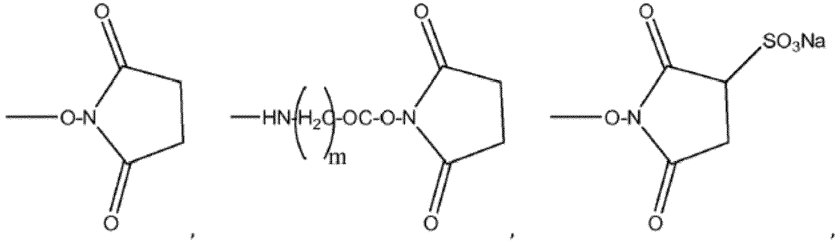 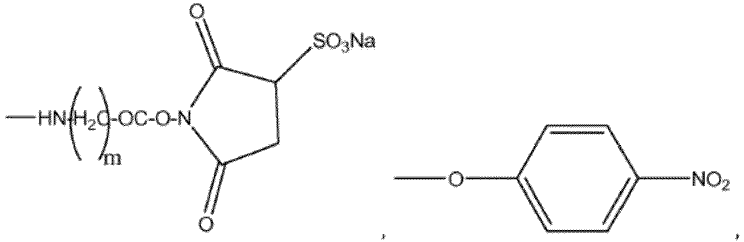 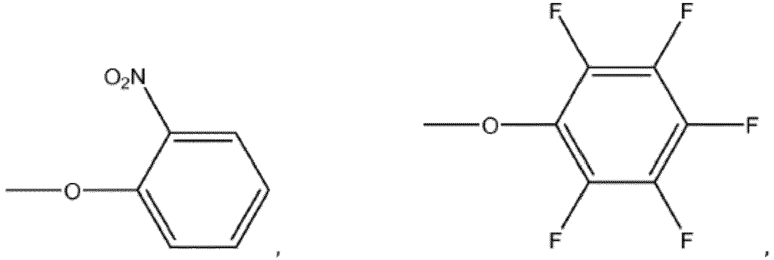 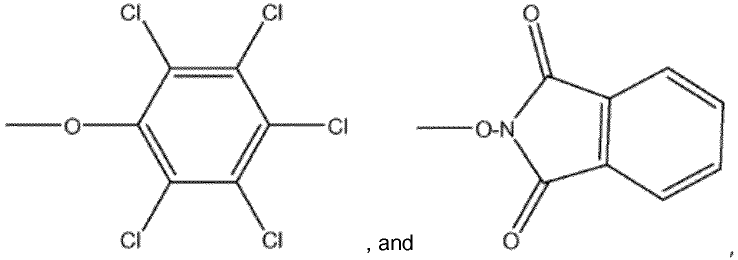
m being an integer between 1 and 10.

8. Use according to claim 7, wherein m is between 1 and 5, advantageously between 1 and 3.

9. Use according to one of claims 5 to 8 wherein the (L)-Gp group is selected from:
-OH and -NH-(CH₂)ₘ-COOH and 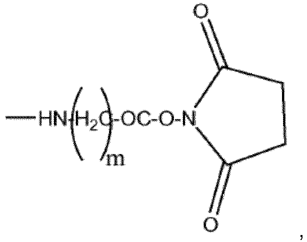
m being between 1 and 10 and preferably m being equal to 1.

10. Use according to one of claims 1 to 9, wherein the derivative of general formula (I) is used in conjugate form with said molecule M, and said conjugate being represented by the following general formula (III): 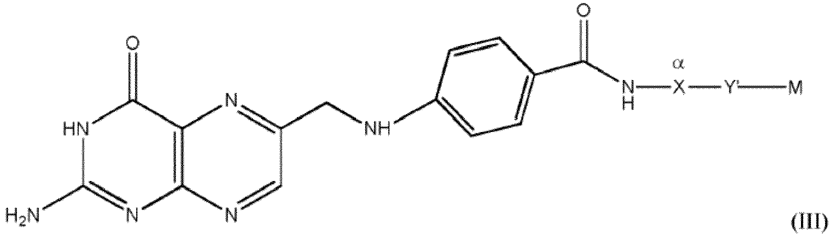
wherein X and M are as defined in one of claims 1 to 9, and wherein Y' is a derivative of the functional group Y after bonding of the derivative of general formula (I) to said molecule M,
said molecule M preferably being a marker molecule Mm.

11. Use according to claim 10, wherein Y' is represented by the following general formula (IV): 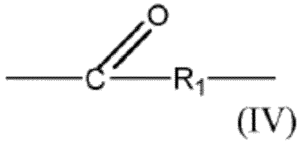 or by the following general formula (V): 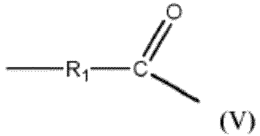
wherein R1 is -NH-, -O-, or -S-, preferably -NH- or -O-, advantageously -NH-;
preferably Y' being represented by general formula (IV).

12. Process for *in vitro* assay of the folate(s) in a sample, such as a biological sample, said assay being a non-radioisotopic competition immunoassay, said process comprising the following steps:
a) bringing into contact, in said biological sample, (i) at least one binding partner of the folate(s), such as an antibody suitable for binding to the folate(s) or such as the folates receptor, with (ii) at least one compound selected from a folate derivative as defined in one of claims 1 to 9 and a conjugate as defined in claim 10 or 11, at least one of said compounds (i) and (ii) being suitable for emitting a signal,
b) possibly leaving a sufficient time interval to enable the competition reaction,
c) measuring the intensity of the signal and deducing from it the folate(s) concentration present in said biological sample by reference to a calibration curve establishing a relationship between the measured signal intensity and folate(s) concentration.

13. Process according to claim 12, **characterised in that** the compound (ii) is a conjugate as defined in claim 10 or 11.

14. *In vitro* diagnostic method intended to determine whether a human or animal patient, preferably human, has a folate(s) deficiency, said method comprising the following steps:
a) in a biological sample, preferably of whole blood, plasma or serum, taken from said patient, assaying the folate(s) by implementing the *in vitro* assay process according to claim 12 or 13, in order to deduce from it the folate(s) concentration in the biological sample
b) comparing said concentration with a threshold value, corresponding to a predetermined folate(s) concentration below which a patient is considered as folate(s) deficient, and
c) if the assayed concentration is lower than said threshold value, deducing from this that the patient has a folate(s) deficiency.

15. Folate derivative of general formula (I), suitable for being used in a non-radioisotopic competition immunoassay: 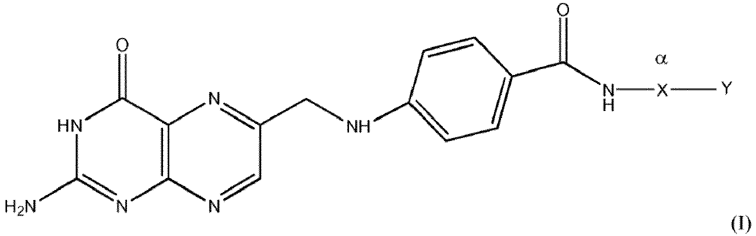 wherein:
- X is as defined in one of claims 1 to 9;
- Y is an electrophilic centre type group or nucleophilic centre type group, preferably an electrophilic centre type group, suitable for enabling the formation of an amide, ester, or thioester bond, preferably amide or ester, advantageously amide, between Y and a functional group carried by a separate molecule M; and wherein:
- if X is a linear and saturated hydrocarbon chain containing a number of carbon atoms equal to 2, Y is not an -(O-CH₂-CH₂)₂-NH₂ group;
- if Y is a nucleophilic centre type group consisting of a primary amine, X comprises a number of carbon atoms different from 6,
- if Y is an electrophilic centre type group, the latter corresponds to the following general formula (II): 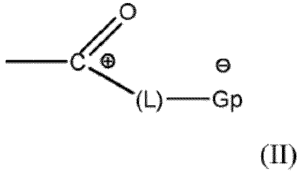
wherein Gp is a leaving group, possibly connected to the carbonyl function by an L arm, Gp being suitable for being dissociated from the electrophilic centre type group in a reaction with a nucleophilic group carried by said separate molecule M, such as a primary amine, and wherein, if L is absent and Gp is -OH, X comprises a number of carbon atoms greater than 4.

16. Folate derivative according to claim 15, wherein Y is a nucleophilic centre type group consisting of a functional group different from a primary amine.

17. Folate derivative according to claim 15, wherein Y is an electrophilic centre type group corresponding to general formula (II) and wherein (L)-Gp is a leaving group selected from: -OH, -NH-(CH₂)ₘ-COOH, -N₃, 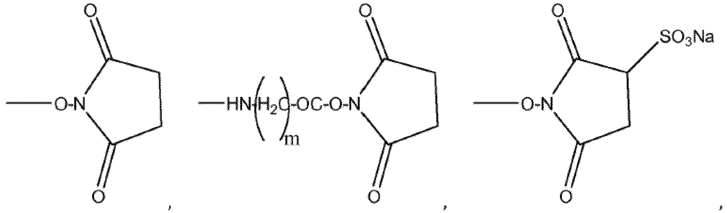 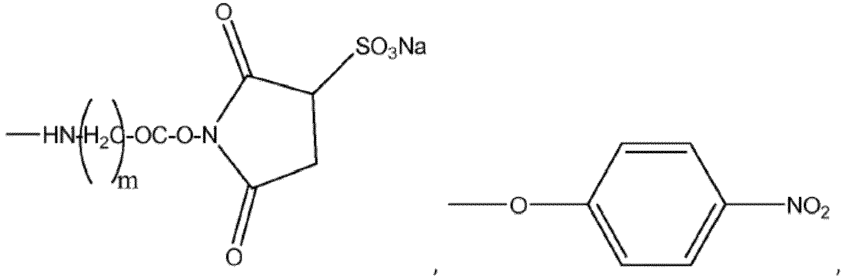 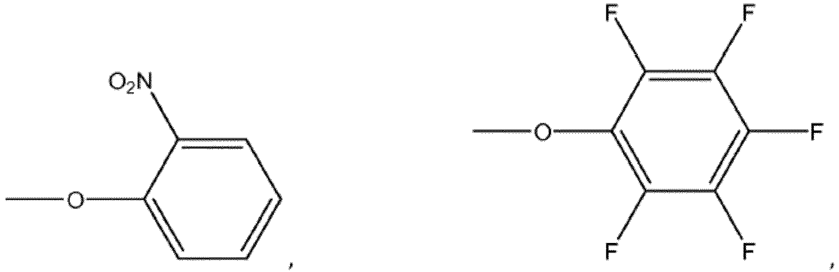 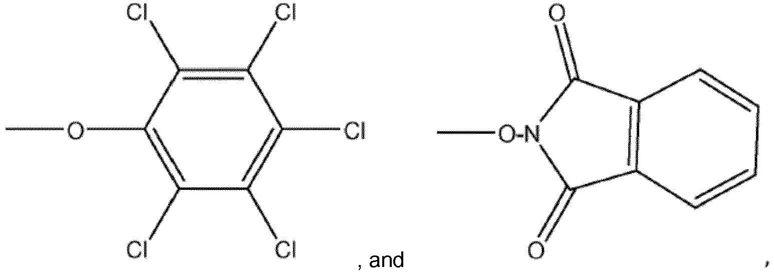 m being an integer between 1 and 10, preferably between 1 and 5, advantageously between 1 and 3, wherein if L is absent and Gp is -OH, X comprises a number of carbon atoms greater than 4.

18. Folate derivative according to claim 17, wherein (L)-Gp is selected from:
-OH and -NH-(CH₂)ₘ-COOH and 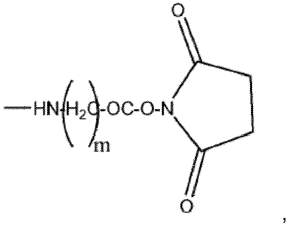
m being between 1 and 10 and preferably m being equal to 1,
wherein if L is absent and Gp is -OH, X comprises a number of carbon atoms greater than 4.

19. Folate derivative according to one of claims 15, 17 or 18, wherein Gp is a group different from an -OH group.

20. Conjugate comprising a folate derivative according to one of claims 15 to 19 and a separate molecule M, said derivative and said molecule M being bound by at least one amide, ester, or thioester bond, preferably an amide or ester bond, advantageously an amide bond, between Y and a functional group carried by said molecule M.

21. Conjugate according to claim 20, wherein said molecule M is a marker molecule Mₘ enabling direct or indirect labelling of said folate derivative.

22. Kit enabling the implementation of the process according to claim 12 or 13, said kit comprising:
- (i) at least one binding partner of the folate(s), such as an antibody suitable to bind to the folate(s) or such as the folates receptor,
- (ii) at least one compound selected from a folate derivative as defined in one of claims 1 to 9 and a conjugate as defined in claim 10 or 11,
at least one of said compounds (i) and (ii) being suitable for emitting a signal, and
- at least one calibration means.
